(19) Europäisches Patentamt
European Patent Office
Office européen des brevets

(11) Veröffentlichungsnummer: **0 278 453 B1**

(12) # EUROPÄISCHE PATENTSCHRIFT

(45) Veröffentlichungstag der Patentschrift: **28.12.94**

(51) Int. Cl.[5]: **C07D 213/81**, C07D 213/82, C07D 417/12, C07D 409/12, C07D 401/12, A61K 31/44

(21) Anmeldenummer: **88101792.5**

(22) Anmeldetag: **08.02.88**

Die Akte enthält technische Angaben, die nach dem Eingang der Anmeldung eingereicht wurden und die nicht in dieser Patentschrift enthalten sind.

(54) **Pyridin-2,4- und 2,5-dicarbonsäureamide, Verfahren zu ihrer Herstellung, Verwendung derselben sowie Arzneimittel auf Basis dieser Verbindungen.**

(30) Priorität: **10.02.87 DE 3703959**

(43) Veröffentlichungstag der Anmeldung: **17.08.88 Patentblatt 88/33**

(45) Bekanntmachung des Hinweises auf die Patenterteilung: **28.12.94 Patentblatt 94/52**

(84) Benannte Vertragsstaaten: **AT BE CH DE ES FR GB GR IT LI LU NL SE**

(56) Entgegenhaltungen:
**EP-A- 0 198 202**

**J. MED. CHEM., Band 10, Nr. 4, Juli 1967, Seiten 706-713; G.J. ATWELL et al.:"Potential antitumor agents. V. Bisquaternary salts"**

**CHEMICAL ABSTRACTS, Band 80, 1974, Seite 407, Nr. 108395y, Columbus, Ohio, US;**

(73) Patentinhaber: **HOECHST AKTIENGESELLSCHAFT**
**D-65926 Frankfurt (DE)**

(72) Erfinder: **Bickel, Martin, Dr.**
**Mittelstedter Weg 3**
**D-6380 Bad Homburg (DE)**
Erfinder: **Brocks, Dietrich, Dr.**
**Goethering 9**
**D-6200 Wiesbaden 42 (DE)**
Erfinder: **Burghard, Harald, Dr.**
**Feldbergstrasse 96c**
**D-6384 Schmitten 3 (DE)**
Erfinder: **Günzler, Volkmar, Dr.**
**Marburger Strasse 2**
**D-3550 Marburg-Cappel (DE)**
Erfinder: **Henke, Stephan, Dr.**
**Sophienruhe 4**
**D-6232 Bad Soden am Taunus (DE)**
Erfinder: **Hanauske-Abel, Hartmut, Dr.**
**Schlossstrasse 35**
**D-6501 Dexheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**LIEBIGS ANNALEN DER CHEMIE, Nr. 5, 1982, Seiten 930-949, Verlag Chemie,Weinheim, DE; E. LANGHALS et al.: "Eine einfache neue Synthese der Fusarinsäureund anderer 5-Alkyl-2-pyridincarbonsäuren"**

Erfinder: **Mohr, Jürgen, Dr.**
**Im Hochgewanne 48**
**D-6718 Grünstadt (DE)**
Erfinder: **Tschank, George, Dr.**
**An den Mühlwegen 34**
**D-6500 Mainz 42 (DE)**

## Beschreibung

Verbindungen, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie $\alpha,\alpha'$-Dipyridyl zu einer Hemmung der $C1_q$-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4-und -2,5-dicarbonsäure effektiv gehemmt wird (K. Mayama et al., Eur. J. Biochem. 138 (1984) 239-245). Diese Verbindungen sind in der Zellkultur allerdings nur in sehr hohen Konzentrationen als Hemmstoffe wirksam (V. Günsler et al. Collagen and Rel. Research 3, 71 1983).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Überraschenderweise wurde nun gefunden, daß die Diamide der Pyridin-2,4- und -2,5-dicarbonsäure ausgezeichnete Hemmstoffe der Kollagenbiosynthese im Tiermodell sind.

Der eigentliche Wirkstoff, die Pyridin-2,4- oder 2,5-Dicarbonsäure entsteht erst durch Hydrolyse der Diamide in der Zelle. Die Diamide können auf Grund ihrer höheren Lipophilie und der Tatsache, daß sie während des Transports überaschenderweise nur sehr langsam hydrolysiert werden bis in die Zellen transportiert werden. Erst hier wird der eigentliche Wirkstoff, Pyridin-2,4 oder -2,5-dicarbonsäure, freigesetzt.

Die Erfindung betrifft somit:

1. Pyridin-2,4- und -2,5-dicarbonsäure-Amide der Formel I

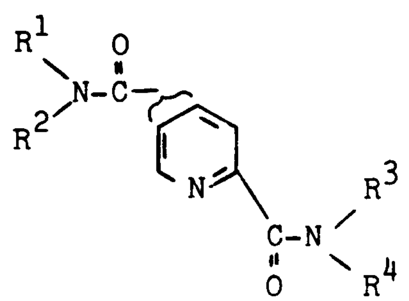

(I)

worin

$R^1$ verzweigtes oder unverzweigtes $C_1$-$C_{12}$-Alkyl, welches gegebenenfalls einfach oder im Falle der $C_2$-$C_{12}$-Alkyleauch mehrfach substituiert ist mit

Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, die im Falle der $C_3$- und $C_4$-Alkylreste auch verzweigt sein können,

Indolyl oder Phenyl, welches seinerseits gegebenenfalls mit Halogen, Nitro, $C_1$-$C_4$-Alkyl oder $C_1$-$C_4$-Alkoxy, 1-, 2-, oder 3-fach substituiert ist, wobei bei Mehrfachsubstitutionen die Substituenten auch voneinander unabhängig verschieden sein können und wobei im Falle der $C_3$- und $C_4$-Alkyle diese auch verzweigt sein können,

oder $R^1$ gesättigtes $C_5$-$C_7$-Cycloalkyl, welches gegebenenfalls benzoanneliert ist,

oder $R^1$ Phenyl, Naphthyl oder ein 5- oder 6-gliedriger aromatischer Ring mit 1, 2 oder 3

Stickstoff- und/oder Sauerstoff- und/oder Schwefelatomen, jeweils gegebenenfalls mit Halogen, Nitro, Cyano, $C_1$-$C_4$-Alkyl oder $C_1$-$C_4$-Alkoxy 1-, 2-, oder 3-fach substituiert, wobei bei Mehrfachsubstitutionen die Substituenten auch voneinander unabhängig verschieden sein können und wobei im Falle der $C_3$-und $C_4$-Alkyle diese auch verzweigt sein können

und

$R^2$ unabhängig von $R^1$ Wasserstoff ist oder eine der Bedeutungen wie für $R^1$ beschrieben hat, wobei $R^2$ auch identisch mit $R^1$ sein kann, und

$R^3$ unabhängig von $R^1$ und $R^2$ Wasserstoff ist oder eine der Bedeutungen für $R^1$ beschrieben hat, wobei $R^3$ auch identisch mit $R^1$ und/oder $R^2$ sein kann und

$R^4$ unabhängig von $R^1$, $R^2$ und $R^3$ Wasserstoff ist oder eine der Bedeutungen wie für $R^1$ beschrieben hat wobei

$R^4$ auch identisch mit $R^1$ und/oder $R^2$ und/oder $R^3$ sein kann

und wobei die Reste $R^1$ und $R^2$ und/oder $R^3$ und $R^4$ auch zusammen mit dem Stickstoffatom einen 5-, 6- oder 7-gliedrigen gesättigten heterocyclischen Ring bilden, wobei der heterocyclische Ring auch ein zweites Stickstoffatom beinhalten kann und wobei der heterocyclische Ring seinerseits mit Phenyl oder Phenyl-$C_1$-$C_3$-Alkyl substituiert sein kann,

bedeutet und deren physiologisch verträgliche Salze, zur Anwendung als Arzneimittel,
ausgenommen die Verbindungen, in denen $R^1 = R^3$ mit Methyl und Brom disubstituiertes Phenyl und $R^2 = R^4$ Wasserstoff bedeutet.

Im Besonderen betrifft die Erfindung Pyridin-2,4- und -2,5-dicarbonsäureamide gemäß Formel I, worin

$R^1$ verzweigtes oder unverzweigtes $C_1$-$C_{12}$-Alkyl welches gegebenenfalls einfach oder im Falle der $C_3$-$C_{12}$-Alkyleauch mehrfach substituiert ist, mit Amino oder $C_1$-$C_3$-Alkoxy, wobei die $C_3$-AlkylReste auch verzweigt sein können und/oder Indolyl und/oder Phenyl, welches substituiert sein kann mit $C_1$-$C_4$-Alkoxy oder Nitro

oder $R^1$ benzoanneliertes Cyclohexyl

oder $R^1$ Phenyl, das gegebenenfalls 1-, 2- oder 3-fach Nitro-Cyano-, Halogen- oder $C_1$-$C_4$-Alkyl substituiert ist, wobei bei Mehrfachsubstitution die Substituenten auch unabhängig voneinander verschieden sein können,

oder $R^1$ 2-, 3- oder 4-Pyridyl, 2- oder 3-Thienyl oder Thiazolyl, wobei diese Heteroatome auch mit $C_1$-$C_4$-Alkylmonosubstituiert sein können

bedeutet und

$R^2$ unabhängig von $R^1$ Wasserstoff ist oder eine der Bedeutungen wie für $R^1$ beschrieben hat, wobei $R^2$ auch identisch mit $R^1$ sein kann und

$R^3$ unabhängig von $R^1$ und $R^2$ Wasserstoff ist oder eine der Bedeutungen wie für $R^1$ beschrieben hat, wobei $R^3$ auch identisch mit $R^1$ und/oder $R^2$ sein kann und

$R^4$ unabhängig von $R^1$, $R^2$ und $R^3$ Wasserstoff ist oder eine der Bedeutungen wie für $R^1$ beschrieben hat, wobei $R^4$ auch identisch mit $R^1$ und/oder $R^2$ und/oder $R^3$ sein kann

bedeutet, sowie deren physiologisch verträgliche Salze, zur Anwendung als Arzneimittel ausgenommen die Verbindungen, in denen $R^1$ = $R^3$ mit Methyl und Brom disubstituiertes Phenyl und $R^2$ = $R^4$ Wasserstoff bedeutet.

Außerdem betriff die Erfindung Pyridin-2,4- und -2,5-dicarbonsäureamide Formel I′,

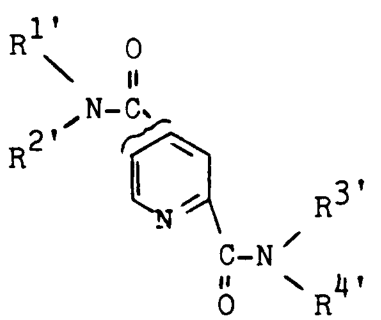

(I')

worin

$R^{1'}$ verzweigtes oder unverzweigtes $C_1$-$C_{12}$-Alkyl, welches gegebenenfalls einfach oder im Falle von $C_2$-$C_{12}$-Alkyl auch mehrfach substituiert ist mit Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, und wobei im Falle der $C_3$- und $C_4$Alkyle diese auch verzweigt sein können Indolyl oder Phenyl, welches seinerseits gegebenenfalls mit Halogen, Nitro, $C_1$-$C_4$-Alkoxy oder $C_1$-$C_4$-Alkyl 1-, 2- oder 3-fach substituiert ist, wobei die genannten $C_3$- und $C_4$-Alkylreste auch verzweigt sein können und wobei bei Mehrfachsubstitutionen die Substituenten auch voneinander unabhängig verschieden sein können

oder $R^{1'}$ gesättigtes $C_5$-$C_7$-Cycloalkyl, welches gegebenenfalls benzoanneliert ist

oder $R^{1'}$ Phenyl, Naphthyl oder ein 5- oder 6-gliedriger aromatischer Ring mit 1, 2 oder 3 Stickstoff- und/oder Sauerstoff- und/oder Schwefelatomen, jeweils gegebenenfalls mit Halogen, Nitro, Cyano, $C_1$-$C_4$-Alkyl oder $C_1$-$C_4$-Alkoxy 1-, 2- oder 3-fach substituiert, wobei bei Mehrfachsubstitutionen die Substituenten auch voneinander unabhängig verschieden sein können und wobei im Falle der $C_3$- und $C_4$-Alkyle diese auch verzweigt sein können,

und

$R^{2'}$ unabhängig von $R^{1'}$ Wasserstoff ist oder eine der Bedeutungen wie für $R^{1'}$ beschrieben hat, wobei $R^{2'}$ auch identisch mit $R^{1'}$ sein kann

$R^{3'}$ unabhängig von $R^{1'}$ Wasserstoff ist oder eine der Bedeutungen wie für $R^{1'}$ beschrieben hat, wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann

und wobei die Reste $R^{1'}$ und $R^{2'}$ und/oder $R^{3'}$ und $R^{4'}$ auch zusammen mit dem Stickstoffatom einen 5-, 6-oder 7-gliedrigen gesättigten heterocyclischen Ring bilden können, wobei der heterocyclische Ring auch ein zweites Stickstoffatom beinhalten kann und wobei der heterocyclische Ring seinerseits mit Phenyl oder Phenyl-$C_1$-$C_3$-alkyl substituiert sein kann,

bedeutet,

sowie deren physiologisch verträgliche Salze, ausgenommen die Verbindungen, in denen $R^{1'}$ = $R^{3'}$ 2-Hydroxyethyl oder 2-(3,4-dimethoxyphenyl)ethyl oder mit Methyl und Brom disubstituiertes Phenyl und $R^{2'}$ = $R^{4'}$ Wasserstoff bedeutet und N,N,N',N'-Tetracyclohexyl-2,5-pyridindicarbonsäurediamid.

Bevorzugt sind Pyridin-2,4- und -2,5-dicarbonsäureamide gemäß Formel I', worin

$R^{1'}$ verzweigtes oder unverzweigtes $C_1$-$C_{12}$-Alkyl, welches gegebenenfalls einfach oder im Falle der $C_2$-$C_{12}$-Alkyleauch mehrfach substituiert ist mit Amino oder $C_1$-$C_3$-Alkoxy, wobei die Alkylreste im Falle der $C_3$-Alkylverbindungen auch verzweigt sein können oder Indolyl oder Phenyl, welches substituiert sein kann mit $C_1$-$C_4$-Alkoxy oder Nitro

oder $R^{1'}$ benzoanneliertes Cyclohexyl

oder $R^{1'}$ Phenyl, welches gegebenenfalls mit 1, 2 oder 3 Cyano-, Halogen-, $C_1$-$C_4$-Alkyl- oder Nitrogruppen substituiert ist wobei bei Mehrfachsubstitutionen die Substituenten auch unabhängig voneinander verschieden sein können

oder $R^{1'}$ 2-, 3- oder 4-Pyridyl, 3-Thienyl oder Thiazolyl,

und

$R^{2'}$ unabhängig von $R^{1'}$, Wasserstoff ist oder eine der Bedeutungen wie für $R^{1'}$ beschrieben hat, wobei $R^{2'}$ auch identisch mit $R^{1'}$ sein kann

$R^{3'}$ unabhängig von $R^{1'}$ und $R^{2'}$ Wasserstoff ist oder eine der Bedeutungen wie für $R^{1'}$ beschrieben hat, wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann

bedeutet,

sowie deren physiologisch verträgliche Salze,

ausgenommen die Verbindungen, in denen $R^{1'}$ = $R^{3'}$ 2-Hydroxyethyl oder 2-(3,4-Dimethoxyphenyl)ethyl oder mit Methyl und Brom disubstituiertes Phenyl und $R^{2'}$ = $R^{4'}$ Wasserstoff bedeutet.

Besonders bevorzugt sind Pyridin-2,4- und -2,5-dicarbonsäure-Amide gemäß Formel I′ nach Anspruch 3, worin

$R^{1'}$ verzweigtes oder unverzweigtes $C_1$-$C_4$-Alkyl, welches substituiert ist mit $C_1$-$C_3$-Alkoxy, wobei die Alkylreste 1 - 3 C-Atome aufweisen und im Falle der $C_3$-Alkylverbindungen auch verzweigt sein können, und

$R^{2'}$ unabhängig von $R^{1'}$ Wasserstoff ist oder eine der Bedeutungen wie für $R^{1'}$ beschrieben hat, wobei $R^2$ auch identisch mit $R^{1'}$ sein kann und

$R^{3'}$ unabhängig von $R^{1'}$ Wasserstoff ist oder eine der Bedeutungen wie für $R^{1'}$ beschrieben hat, wobei $R^3$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann, sowie

deren physiologisch verträgliche Salze.

Die zuletzt genannte Gruppe von Verbindungen besitzt u. a. bei der oralen Anwendung eine besondere Wirksamkeit, wie auch die ganz besonders bevorzugten Pyridin-2,4- und -2,5-dicarbonsäureamide gemäß Formel I′ nach Anspruch 3, worin $R^{1'}$ und $R^{3'}$ eine Isopropoxypropyl-Gruppe ist und $R^{2'}$ und $R^{4'}$ Wasserstoff ist, (wie z. B. Pyridin-2,5-dicarbonsäure-N,N′-di(3-isopropoxy-propyl)amid, Bsp. 3) Pyridin-2,4-dicarbonsäure-bis-3-isopropoxypropylamid (Bsp. 20))und Pyridin-2,4- und -2,5-dicarbonsäureamide gemäß Formel I′ nach Anspruch 3, worin $R^{1'}$ und $R^{3'}$ eine 2-Methoxyethyl-Gruppe ist und $R^{2'}$ und $R^{4'}$ Wasserstoff ist, wie z. B. N,N′-Bis(2-methoxy-ethyl)pyridin-2,4-dicarbonsäureamid (Bsp. 19), sowie deren physiologisch verträgliche Salze.

Unter Halogen werden Fluor, Chlor, Brom und Jod, unter Aryl Phenyl und Naphthyl und unter Heteroaryl 5- und 6-gliedrige aromatische Ringe mit 1, 2 oder 3 Stickstoff- und/oder Sauerstoff- und/oder Schwefelatomen verstanden, die gegebenenfalls noch benzoanneliert sein können; insbesondere handelt es sich bei den Heteroarylresten um Pyridyl-, Pyridazyl-, Pyrimidyl-, Pyrazyl-, 1,3,5-Triazyl-, Pyrolyl-, Pyrazolyl-, Imidazolyl-, Triazolyl-, Thienyl-, Oxazolyl-und Thiazolyl-Reste und gegebenenfalls deren benzoannelierte Verbindungen.

"Mehrfach substituiert" bedeutet im Vorstehenden und Folgenden, daß mindetens 2 höchstens alle in den Alkylresten vorhandenen Wasserstoffatome durch die genannten Substituenten ersetzt sind. Bevorzugt handelt es sich dabei um einen Substituenten pro Methyl- bzw. Methylengruppe.

Bei Mehrfachsubstitutionen können die Substituenten auch voneinander unabhängig verschieden sein.

Weiterhin betrifft die Erfindung ein Verfahren zur Herstellung von Verbindungen der Formel I′, das dadurch gekennzeichnet ist, daß man
eine Verbindung der Formel II

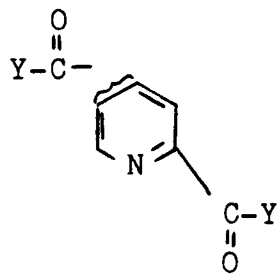

(II)

mit einer Verbindung der Formel III

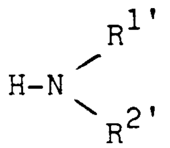

(III)

umsetzt, wobei $R^{1'} = R^{3'}$, $R^{2'} = R^{4'}$ die zu Formel I′ angegebenen Bedeutungen haben und Y Halogen oder Hydroxy ist oder zusammen mit der Carbonylgruppe einen Aktivester oder ein gemischtes Anhydrid bildet, und die Reaktionsprodukte gegebenenfalls in ihre physiologisch verträglichen Salze überführt.

Weiterhin betrifft die Erfindung ein Verfahren zur Herstellung von Verbindungen der Formel I′, das dadurch gekennzeichnet ist, daß man eine Verbindung der Formel II′

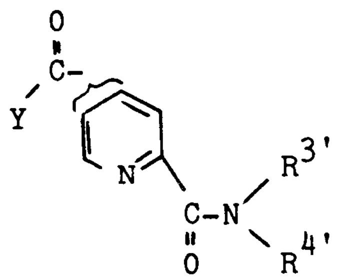

II'

mit einer Verbindung der Formel III

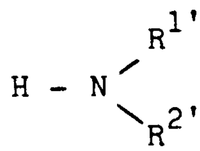

III

umsetzt, wobei $R^{1'}$, $R^{2'}$, $R^{3'}$ und $R^{4'}$ die zu Formel I' angegebenen Bedeutungen haben und Y Halogen oder Hydroxy ist oder zusammen mit der Carbonylgruppe einen Aktivester oder ein gemischtes Anhydrid bildet, und die Reaktionsprodukte gegebenenfalls in ihre physiologisch verträglichen Salze überführt.

Im folgenden wird die Herstellung von Verbindungen gemäß Formel I und die Herstellung der dafür benötigten Ausgangssubstanzen - sofern sie nicht käuflich sind - näher beschrieben.

Die Herstellung der erfindungsgemäßen Verbindungen gelingt am einfachsten dadurch, daß die beiden Komponenten, das Pyridin-Derivat gemäß Formel (II) bzw. Formel (II') und das Amin gemäß Formel (III) in äquimolaren Mengen oder bis zu einem etwa 5-fachen Überschuß an III, vermengt werden und bei Temperaturen zwischen -30 bis 150 °C, bevorzugt bei 20 bis 100 °C bis zur Beendigung der Reaktion umgesetzt werden. Die Beendigung der Reaktion läßt sich mittels Dünnschichtchromatographie (DC-Kontrolle) bestimmen. Eine Variante dieses Verfahrens besteht darin, daß man in einem geeigneten Lösungsmittel, wie Diäthyläther oder Dimethoxyäthan oder Tetrahydrofuran, chlorierten Kohlenwasserstoffen wie Methylenchlorid, Chloroform, Tri-oder Tetrachloräthylen, Benzol, Toluol oder auch polaren Lösungsmitteln wie Dimethylformamid oder Aceton oder Dimethylsulfoxid arbeitet. Auch hier kann ein Überschuß von Amin gemäß Formel (III), der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130 °C besonders bevorzugt sind.

Ebenso kann die Umsetzung über ein gemisches Anhydrid wie Chlorameisensäureethylester oder über einen aktivierten Ester wie Paranitrophenylester (Y = $ClCH_2$-COO oder $NO_2$-$C_6H_4$-O) erfolgen. Entsprechende Methoden sind in Houben-Weyl, Methoden der Organischen Chemie, Band XV/2, Seiten 169-183 (gemischte Anhydridmethode) bzw. Seiten 13ff. (Aktivestermethode), vierteAuflage, Georg Thieme Verlag, Stuttgart 1974, beschrieben.

Gegebenenfalls kann die Umsetzung auch in Gegenwart von Basen erfolgen. Als zusätzliche Basen kommen anorganische Säurefänger wie Carbonate oder Hydrogencarbonate z.B. Natrium- oder Kaliumcarbonat oder Natrium- oder Kaliumhydrogencarbonat, oder organische Säurefänger wie tertiäre Amine, wie Triäthylamin, Tributylamin, Äthyldiisopropylamin oder heterocyclische Amine wie N-Alkylmorpholin, Pyridin, Chinolin oder Dialkylaniline in Betracht.

Bevorzugt erfolgt die Umsetzung der Verbindungen gemäß Formel (II) bzw. Formel (II') mit Aminen gemäß Formel (III) unter Zusatz eines wasserabspaltenden Mittels wie Dialkylcarbodiimid, wobei die Alkylreste 1 bis 8 C-Atome aufweisen, die im Falle der $C_3$-$C_8$-Verbindungen auch verzweigt oder cyclisch sein können; bevorzugt wird Dicyclohexylcarbodiimid eingesetzt. Eine entsprechende Methode ist in Houben-Weyl, Bd. XV/2, Seiten 103-111, Methoden der Organischen Chemie, 4. Aufl., Georg Thieme Verlag, Stuttgart, 1974, beschrieben.

Gegebenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:

Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III) können, soweit sie nicht käuflich sind, einfach synthetisiert werden (z.B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister, S. 822).

Die Ausgangsverbindungen der Formel (II) erhält man beispielsweise durch Umsetzung von Pyridin-2,4- oder -2,5-dicarbonsäure zu dem entsprechenden Pyridin-2,4- oder -2,5-dicarbonsäurehalogenid, bevorzugt -chlorid (nach literaturbekannten Verfahren, z.B. Organikum, Organisch Chemisches Grundpraktikum, 15. Aufl., VEB Deutscher Verlag der Wissenschaften, 1976, S. 595 ff), welches dann mit einem geeigneten Alkohol, z.B. Paranitrobenzylalkohol zu dem entsprechenden Aktivester umgesetzt wird. Ebenso kann die Pyridin-2,4- oder -2,5-dicarbonsäure auch zunächst unter Zusatz einer geeigneten Carbonsäure oder Carbonsäureester wie Chlorameisensäureethylester in ein gemischtes Anhydrid überführt werden, welches dann mit den Aminen (III) zu den erfindungsgemäßen Produkten umgesetzt wird. Eine entsprechende Methode ist z.B. in Houben-Weyl, Methoden der organischen Chemie, Band XV/2, Seiten 169-183, vierte Auflage, 1974, Georg Thieme Verlag Stuttgart, beschrieben.

Die Ausgangsverbindungen der Formel (II′) können beispielsweise folgendermaßen synthetisiert werden:
Umsetzung eines Pyridin-2,4- oder -2,5-Dicarbonsäurehalogenids, vorzugsweise des -chlorids, mit Benzylalkohol zum Pyridin-2,4- oder -2,5-dicarbonsäurebenzylester; anschließende selektive Verseifung des Esters in 2-Position (z.B. in Gegenwart in Gegenwart eines Kupferkatalysators, Acta Helv. 44, 1963, S. 637, überführen der freien Säure in 2-Stellung in das Säurehalogenid, Umsetzung mit einer Verbindung der Formel (III) zum Pyridin-4- oder 5-Carbonsäurebenzylester-2-carbonsäure-amid, hydrogenolytischer Abspaltung der verbliebenen Benzylschutzgruppe (z.B. mit $H_2$/Pt, s.Houben-Weyl Bd. IV/1c (1980), s. 381-82) und anschließender Überführung der freien Säure in 4- bzw. 5-Position des Pyridinrings in das Säurehalogenid (II′).

Das Pyridin-2,4 oder -2,5-dicarbonsäurehalogenid kann nach bekannten Methoden, z.B. durch Umsetzung von Pyridin-2,4-oder -2,5-dicarbonsäure mit Phosphortrihalogenid erhalten werden (s. z.B. Organikum, Organisch Chemisches Grundpraktikum, 15. Aufl., VEB Deutscher Verlag der Wissenschaften, 1976, S. 527 und 595ff).

Die erfindungsgemäßen Verbindungen der Formel I bzw. I′ besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-$NC_1$-Konzentrationen in der Leber von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemäße Verbindung verabreicht wurde

gemessen (diese Testmethode wird beschrieben von Rouiller, C., experimental toxic injury of the liver; in The Liver, C. Rouiller, Vol. 2, S. 335-476, New York, Academic Press, 1964).

Die pharmakologische Wirksamkeit der erfindungsgemäßen Substanzen wurde in zwei Versuchsreihen (s. Tabelle 1 und 2) untersucht. Dabei zeigte sich eine deutliche Hemmung der Prolin- und Lysinhydroxylase.

Tabelle 1

| Substanz aus Bsp. | Dosierung | Hydroxyprolin µg/mg Leber | Prokollagen III Peptid ng/ml | 7s Kollagen ng/ml | Typ IV Kollagen NC1 ng/ml |
|---|---|---|---|---|---|
| 2 | | 0.407 | 56.9 | 217.1 | 110.9 |
| $CCl_4$-Kontrolle | | 0.733 | 81.5 | 477.5 | 192.1 |
| Kontrolle | | 0.143 | 21.4 | 29.1 | 24.9 |

Tabelle 2

| Substanz aus Bsp. | Dosierung | Hydroxyprolin µg/mg Leber | Prokollagen-III-Peptid ng/ml | 7s-Kollagen ng/ml | Typ-IV-Kollagen-NC1 ng/ml |
|---|---|---|---|---|---|
| 3 | 2 x 25 mg | 0.534 | 48.5 | 278.1 | 181.4 |
| $CCl_4$-Kontrolle | | 0.773 | 73.9 | 308.7 | 168.4 |
| Kontrolle | | 0.289 | 11.1 | 22.8 | 23.5 |

Die Verbindungen der Formel I bzw. I' können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Die pharmazeutischen Präparate können in fester Form, z.B. als Tabletten, Dragees, Suppositorien oder Kapseln; in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konservierungs-, Stabilisierungs-, Netz-oder Emulgiermittel, Salze zur Veränderung des osmotischen Drucks oder Puffer. Sie können auch noch andere therapeutisch wirksame Stoffe enthalten.

Im folgenden wird die Erfindung anhand von Beispielen näher erläutert:

Beispiele

1. Pyridin-2,5-dicarbonsäure-N,N'-diethylamid

100 g Pyridin-2,5-dicarbonsäure werden in 600 ml trockenem Methylenchlorid vorgelegt und mit 800 ml frisch frisch destilliertem Thionylchlorid und 4 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 108 g Ethylamin gelöst in 1000 ml Methylenchlorid bei -30 bis -20 °C zugetropft. Man läßt die Mischung langsam auf Raumtemperatur erwärmen, rührt über Nacht bei Raumtemperatur und filtriert vom ausgefallenen Produkt ab. Die Mutterlauge wird mit Natriumbicarbonat Lösung gewaschen und die organische Phase nach dem Trocknen vom Lösungsmittel befreit. die vereinigten Produkte werden aus Essigester umkristallisiert.

Schmelzpunkt 182 °C Ausbeute 114 g

2. Pyridin-2,4-dicarbonsäure-N,N'-diethylamid

100 g Pyridin-2,4-dicarbonsäure werden analog dem Beispiel 1 umgesetzt und entsprechend aufgearbeitet.
Schmelzpunkt 117 °C Ausbeute 135 g
3. Pyridin-2,5-dicarbonsäure-N,N′-di(3-isopropoxy-propyl)amid
10 g Pyridin-2,5-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 17,5 g 3-Isopropoxypropylamin gelöst in 200 ml Methylenchlorid bei -30 bis -20 °C zugetropft. Man läßt die Mischung langsam auf Raumtemperatur erwärmen, rührt über Nacht bei Raumtemperatur und wäscht die reaktionsmischung mit Natriumbicarbonat Lösung und befreit die organische Phase nach dem Trocknen vom Lösungsmittel. Das Produkt wird über Kieselgel chromatographiert.
Schmelzpunkt 92 °C Ausbeute 8,5 g
4. Pyridin-2,5-dicarbonsäure-N,N′-di(1,2,3,4-tetrahydronapht-1-yl)amid
10 g Pyridin-2,5-dicarbonsäure werden gemäß Beispiel 3 in das Säurechlorid überführt und dann mit 22,0 g 1,2,3,4-Tetrahydro-1-naphthylamin umgesetzt. Zur Reinigung wird das Produkt mit Ethanol ausgekocht.
Schmelzpunkt 206 °C Ausbeute 14.8 g
5. Pyridin-2,5-dicarbonsäure-N,N′-dibenzylamid
10 g Pyridin-2,5-dicarbonsäure werden gemäß Beispiel 3 zunächst in das Säurechlorid überführt und anschließend mit 16.04 g Benzylamid umgesetzt. Zur Reinigung wird das Produkt nach dem Waschen mit Bicarbonatlösung über Kieselgel chromatographiert und anschließend aus Ethanol umkristallisiert.
Schmelzpunkt 188-89 °C Ausbeute 2.5 g
6. Pyridin-2,5-dicarbonsäure-N,N′-diisopropylamid
10 g Pyridin-2,5-dicarbonsäure werden gemäß Beispiel 3 in das Säurechlorid überführt und dann mit 8.85 g wasserfreiem Isopropylamin umgesetzt. Zur Reinigung wird das Produkt nach dem Waschen mit Bicarbonatlösung über Kieselgel chromatographiert und aus Ethanol umkristallisiert.
Schmelzpunkt 175-6 °C Ausbeute 3.9 g
7. Pyridin-2,5-dicarbonsäure-N,N′-dipyrid-(2)-ylamid
10 g Pyridin-2,5-dicarbonsäure werden gemäß Beispiel 3 in das Säurechlorid überführt und anschließend mit 14.1 g 2-Aminopyridin umgesetzt. Zur Reinigung wird nach dem Waschen des Produktes mit Bicarbonatlösung über Kieselgel chromatographiert und aus Ethanol umkristallisiert.
Schmelzpunkt 216-17 °C Ausbeute 2.4 g
8. Pyridin-2,5-dicarbonsäure-N,N′-di(3-phenylpropyl)amid
10 g Pyridin-2,5-dicarbonsäure werden gemäß Beispiel 3 in das Säurechlorid überführt und anschließend mit 17 g 3-Phenylpropylamin umgesetzt. Zur Reinigung wird das Produkt aus Ethanol umkristallisiert.
Schmelzpunkt 139 °C Ausbeute 13.6 g
9. Pyridin-2,5-dicarbonsäure-N,N′-di(1-methoxy-prop-2-yl)amid
10 g Pyridin-2,5-dicarbonsäure werden gemäß Beispiel 3 in das Säurechlorid überführt und anschließend mit 10.7 g 2-Amino-1-methoxy-propan umgesetzt. Das Produkt wird zur Reinigung über Kieselgel mit Essigester chromatographiert.
Schmelzpunkt 112-113 °C Ausbeute 6.4 g
10. Pyridin-2,5-dicarbonsäure-N,N′-di(2-methoxy-ethyl)amid
10 g Pyridin-2,5-dicarbonsäure werden gemäß Beispiel 3 in das Säurechlorid überführt und anschließend mit 9 g 2-Methoxy-ethylamin umgesetzt. Zur Reinigung wird das Produkt über Kieselgel mit einer Mischung aus Methylenchlorid und Aceton chromatographiert.
Schmelzpunkt 97 °C Ausbeute 2.2 g
11. Pyridin-2,4-dicarbonsäure-N,N′-di(thiazol-2-yl)amid
1.02 g Pyridin-2,4-dicarbonsäure-di(4-nitrophenyl)ester werden in 30 ml trockenem Dimethylformamid gelöst und mit 0.5 g 2-Aminothiazol und 0.45 ml Triethylamin drei Stunden bei Raumtemperatur umgesetzt. Die Reaktionsmischung wird in Diethylether aufgenommen und mehrfach mit Wasser gewaschen. Die wäßrige Phase wird einmal mit Essigester extrahiert, die organischen Phasen vereinigt, getrocknet und über Kieselgel mit einer 4:1 Mischung von Toluol und Essigester chromatographiert.
Schmelzpunkt 249 °C Ausbeute 0.176 g
12. N,N′-Bis(3-dibutylamino-propyl)-pyridin-2,5-dicarbonsäure
amid 5 g Pyridin-2,5-dicarbonsäure werden gemäß Beispiel 3 in das Säurechlorid überführt und mit 11.2 g N,N′-Dibutyl-1,3-propandiamin in Methylenchlorid umgesetzt. Die Reaktionsmischung wird mit Natriumbicarbonatlösung verrührt, die Phasen werden getrennt und die wäßrige Phase wird mit Methylenchlorid

extrahiert. Die vereinigten organischen Phasen werden getrocknet und vom Lösungsmittel befreit. Das Produkt wird in Aceton aufgenommen und mit Oxalsäure versetzt. Das gebildete Oxalat wird zuerst aus Essigester-Methanol und ein zweites Mal aus Isopropanol-Aceton umkristallisiert.
Schmelzpunkt 139 °C Ausbeute 8.2 g

13. N,N'-Bis(2-(2-methoxyphenyl)ethyl)-pyridin-2,5-dicarbonsäureamid

5 g Pyridin-2,5-dicarbonsäure werden gemäß Beispiel 3 in das Säurechlorid überführt und mit 9.1 g 2-(2-Methoxyphenyl)ethylamin in Methylenchlorid umgesetzt. Die Reaktionsmischung wird mit Natriumbicarbonatlösung verrührt, die Phasen werden getrennt und die wäßrige Phase wird mit Methylenchlorid extrahiert. Die vereinigten organischen Phasen werden getrocknet und vom Lösungsmittel befreit. Das Produkt wird zweimal aus Essigester umkristallisiert und mit Diethylether gewaschen.
Schmelzpunkt 139-40 °C Ausbeute 2.5 g

14. N,N'-Bis(4-methyl-thien-3-yl)-pyridin-2,5-dicarbonsäureamid

5 g Pyridin-2,5-dicarbonsäure werden gemäß Beispiel 3 in das Säurechlorid überführt und mit 6.8 g 3-Amino-4-methylthiophen in Methylenchlorid umgesetzt. Die Reaktionsmischung wird mit Natriumbicarbonatlösung verrührt, die Phasen werden getrennt und die wäßrige Phase wird mit Methylenchlorid extrahiert. Die vereinigten organischen Phasen werden getrocknet und vom Lösungsmittel befreit. Das Produkt wird zweimal aus Essigester-Methanol umkristallisiert.
Schmelzpunkt 209 °C Ausbeute 3.0 g

15. N,N'-Bis(2-indol-3-yl)prop-2-yl)-pyridin-2,5-dicarbonsäureamid

5 g Pyridin-2,5-dicarbonsäure werden gemäß Beispiel 3 in das Säurechlorid überführt und mit 10.4 g $\alpha,\alpha$-Dimethyl-tryptamin in Methylenchlorid umgesetzt. Die Reaktionsmischung wird mit Natriumbicarbonatlösung verrührt, die Phasen werden getrennt und die wäßrige Phase wird mit Methylenchlorid extrahiert. Die vereinigten organischen Phasen werden getrocknet und vom Lösungsmittel befreit.
Schmelzpunkt 129-30 °C Ausbeute 1.0 g

16. N,N'-Bis(dodecyl)-pyridin-2,5-dicarbonsäureamid

5 g Pyridin-2,5-dicarbonsäure werden gemäß Beispiel 3 in das Säurechlorid überführt und mit 11.1 g Dodecylamin in Methylenchlorid umgesetzt. Die Reaktionsmischung wird mit Natriumbicarbonatlösung verrührt, die Phasen werden getrennt und die wäßrige Phase wird mit Methylenchlorid extrahiert. Die vereinigten organischen Phasen werden getrocknet und vom Lösungsmittel befreit.
Schmelzpunkt 122 °C Ausbeute 3.8 g

17. N,N'-Bis(2-(4-nitrophenyl)ethyl)-pyridin-2,5-dicarbonsäureamid

2 g Pyridin-2,5-di(4-nitrophenyl)carbonsäureester werden in 60 ml Dimethylformamid vorgelegt, mit 4 ml Triethylamin und 1.98 g 2-(4-Nitrophenyl)ethylamin versetzt und drei Stunden bei Raumtemperatur gerührt. Die Reaktionsmischung wird mit Essigester versetzt mehrfach mit Wasser und mit Natriumbicarbonatlösung gewaschen. Die organische Phase wird getrocknet und vom Lösungsmittel befreit. Das Produkt wird zweimal aus Essigester umkristallisiert und mit Diethylether gewaschen.
Schmelzpunkt 211-12 °C Ausbeute 1.1 g

18. N,N'-Bis(2-cyanophenyl)pyridin-2,5-dicarbonsäureamid

5 g Pyridin-2,5-dicarbonsäure werden gemäß Beispiel 3 in das Säurechlorid überführt und mit 7.1 g 2-Aminobenzonitril in Methylenchlorid umgesetzt. Die Reaktionsmischung wird mit Natriumbicarbonatlösung verrührt, die Phasen werden getrennt und die wäßrige Phase wird mit Methylenchlorid extrahiert. Die vereinigten organischen Phasen werden getrocknet und vom Lösungsmittel befreit. Der Rückstand wird mit Diethylether verrührt, abgesaugt und mit Essigester/Methanol ausgekocht und nach dem Abkühlen abgesaugt und mit Diethylether gewaschen.
Schmelzpunkt 252 °C Ausbeute 1.67 g

19. N,N'-Bis(2-methoxy-ethyl)pyridin-2,4-dicarbonsäureamid

10 g Pyridin-2,4-dicarbonsäure werden gemäß Beispiel 3 in das Säurechlorid überführt und mit 8.98 g 2-Methoxyethylamin in Methylenchlorid umgesetzt. Die Reaktionsmischung wird mit Natriumbicarbonatlösung verrührt, die organische Phase wird abgetrennt, getrocknet und vom Lösungsmitel befreit. Der Rückstand wird über Kieselgel mit einer 3:1 Mischung von Essigester und Methanol chromatographiert. Das resultierende Öl wird mit Essigester/Petrolether verrührt und nach Kristallisation abgesaugt.
Schmelzpunkt 86 °C Ausbeute 0.8 g

20. Pyridin-2,4-dicarbonsäure-bis-3-isopropoxypropylamid

10 g Pyridin-2,4-dicarbonsäure werden in 60 ml trockenem Methylenchlorid vorgelegt und mit 80 ml frisch destilliertem Thionylchlorid und 2 ml trockenem Dimethylformamid versetzt. Die Mischung wird drei Stunden unter Rückfluß gekocht und anschließend wird das überschüssige Thionylchlorid und das Methylenchlorid abdestilliert und der Rückstand einmal mit trockenem Toluol abgeraucht. Zu der Reaktionsmischung wird eine Lösung von 17,5 g 3-Isopropoxypropylamin gelöst in 200 ml Methylenchlorid bei -30 bis -20 °C zugetropft. Man läßt die Mischung langsam auf Raumtemperatur erwärmen, rührt über Nacht bei Raumtemperatur und wäscht die Reaktionsmischung mit Natriumbicarbonat Lösung und befreit die organische Phase nach dem Trocknen vom Lösungsmittel. Das Produkt wird über Kieselgel chromatographiert.
Schmelzpunkt 49 °C Ausbeute 12,9 g

21. Pyridin-2-(3-isopropoxypropyl)carbonsäureamid-5-(3-(N,N-dibutylamino)propyl)carbonsäureamid

1 g Pyridin-5-carbonsäure-2-(3-isopropoxypropyl)carbonsäureamid werden in 20 ml Thionylchlorid unter Rückfluß gekocht bis eine klare Lösung entstanden ist. Man läßt eine Stunde bei Raumtemperatur stehen, destilliert das Thionylchlorid ab und tropft 0,7 g N-N-Dibutyl-1,3-propandiamin in 30 ml Methylenchlorid zu. Die Reaktionsmischung wird 30 min bei Raumtemperatur gerührt, vom Lösungsmittel befreit und über Kieselgel mit einer 1:1 Mischung von Essigester und Methanol chromatographiert.
Ausbeute 0.22 g Öl

**Patentansprüche**

**Patentansprüche für folgende Vertragsstaaten : AT, BE, CH, DE, FR, GB, IT, LI, LU, NL, SE**

**1.** Pyridin-2,4- und -2,5-dicarbonsäure-Amide der Formel I

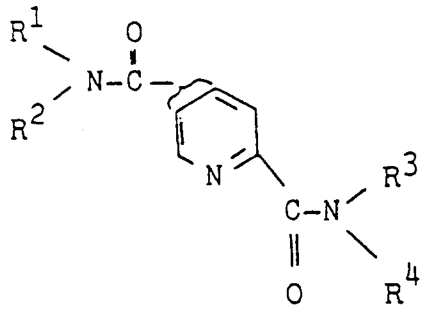

(I)

worin

$R^1$ verzweigtes oder unverzweigtes $C_1$-$C_{12}$-Alkyl, welches gegebenenfalls einfach oder im Falle der $C_2$-$C_{12}$-Alkyleauch mehrfach substituiert ist mit

Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, die im Falle der $C_3$- und $C_4$-Alkylreste auch verzweigt sein können, Indolyl oder Phenyl, welches seinerseits gegebenenfalls mit Halogen, Nitro, $C_1$-$C_4$-Alkyl oder $C_1$-$C_4$-Alkoxy, 1-, 2-, oder 3-fach substituiert ist, wobei bei Mehrfachsubstitutionen die Substituenten auch voneinander unabhängig verschieden sein können und wobei im Falle der $C_3$- und $C_4$-Alkyle diese auch verzweigt sein können,

oder $R^1$ gesättigtes $C_5$-$C_7$-Cycloalkyl, welches gegebenenfalls benzoanneliert ist,

oder $R^{1'}$ Phenyl, Naphthyl oder ein 5- oder 6-gliedriger aromatischer Ring mit 1, 2 oder 3 Stickstoff- und/oder Sauerstoff- und/oder Schwefelatomen, jeweils gegebenenfalls mit Halogen, Nitro, Cyano, $C_1$-$C_4$-Alkyl oder $C_1$-$C_4$-Alkoxy 1-, 2-, oder 3-fach substituiert, wobei bei Mehrfachsubstitutionen die Substituenten auch voneinander unabhängig verschieden sein können und wobei im Falle der $C_3$- und $C_4$-Alkyle diese auch verzweigt sein können

und

$R^2$ unabhängig von $R^1$ Wasserstoff ist oder eine der Bedeutungen wie für $R^1$ beschrieben hat, wobei $R^2$ auch identisch mit $R^1$ sein kann und

$R^3$ unabhängig von $R^1$ und $R^2$ Wasserstoff ist oder eine der Bedeutungen wie für $R^1$ beschrieben hat, wobei $R^3$ auch identisch mit $R^1$ und/oder $R^2$ sein kann und

$R^4$ unabhängig von $R^1$ $R^2$ und $R^3$ Wasserstoff ist oder eine der Bedeutungen wie für $R^1$ beschrieben hat, wobei R auch identisch mit $R^1$ und/oder $R^2$ und/oder $R^3$ sein kann

und wobei die Reste $R^1$ und $R^2$ und/oder $R^3$ und $R^4$ auch zusammen mit dem Stickstoffatom einen 5-, 6- oder 7-gliedrigen gesättigten heterocyclischen Ring bilden können, wobei der heterocyclische Ring auch ein zweites Stickstoffatom beinhalten kann und wobei der heterocyclische Ring seinerseits mit Phenyl oder Phenyl-$C_1$-$C_3$-Alkyl substituiert sein kann,

bedeutet und deren physiologisch verträgliche Salze, zur Anwendung als Arzneimittel, ausgenommen die Verbindungen, in denen $R^1 = R^3$ mit Methyl und Brom disubstituiertes Phenyl und $R^2 = R^4$ Wasserstoff bedeutet.

**2.** Pyridin-2,4- und -2,5-dicarbonsäure-Amide nach Anspruch 1 gemäß Formel I, worin

$R^1$ verzweigtes oder unverzweigtes $C_1$-$C_{12}$-Alkyl welches gegebenenfalls einfach oder im Falle der $C_3$-$C_{12}$-Alkyleauch mehrfach substituiert ist, mit Amino oder $C_1$-$C_3$-Alkoxy wobei die $C_3$-AlkylReste auch verzweigt sein können und/oder Indolyl und/oder Phenyl, welches substituiert sein kann mit $C_1$-$C_4$-Alkoxy oder Nitro

oder $R^1$ benzoanneliertes Cyclohexyl

oder $R^1$ Phenyl, das gegebenenfalls 1-, 2- oder 3-fach Nitro-Cyano-, Halogen- oder $C_1$-$C_4$-Alkyl substituiert ist, wobei bei Mehrfachsubstitutionen die Substituenten auch unabhängig voneinander verschieden sein können

oder $R^1$ 2-, 3- oder 4-Pyridyl, 2- oder 3-Thienyl oder Thiazolyl wobei diese Heteroaromaten auch mit $C_1$-$C_4$-Alkyl monosubstituiert sein können

bedeutet und

$R^2$ unabhängig von $R^1$ Wasserstoff ist oder eine der Bedeutungen wie für $R^1$ beschrieben hat, wobei $R^2$ auch identisch mit $R^1$ sein kann und

$R^3$ unabhängig von $R^1$ und $R^2$ Wasserstoff ist oder eine der Bedeutungen wie für $R^1$ beschrieben hat, wobei $R^3$ auch identisch mit $R^1$ und/oder $R^2$ sein kann und

$R^4$ unabhängig von $R^1$, $R^2$ und $R^3$ Wasserstoff ist oder eine der Bedeutungen wie für $R^1$ beschrieben hat, wobei $R^4$ auch identisch mit $R^1$ und/oder $R^2$ und/oder $R^3$ sein kann

bedeutet, sowie deren physiologisch verträgliche Salze, zur Anwendung als Arzneimittel ausgenommen die Verbindungen, in denen $R^1 = R^3$ mit Methyl und Brom disubstituiertes Phenyl und $R^2 = R^4$ Wasserstoff bedeutet.

**3.** Pyridin-2,4- und -2,5-dicarbonsäure-Amide der Formel I',

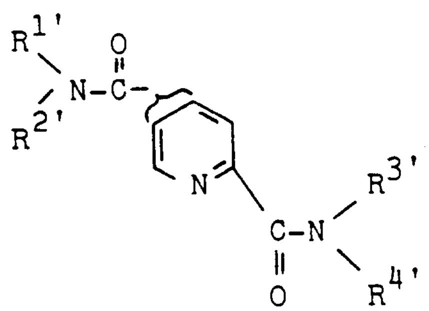

(I')

worin

$R^{1'}$ verzweigtes oder unverzweigtes $C_1$-$C_{12}$-Alkyl, welches gegebenenfalls einfach oder im Falle von $C_2$-$C_{12}$-Alkyl auch mehrfach substituiert ist mit Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, und wobei im Falle der $C_3$- und $C_4$Alkyle diese auch verzweigt sein können, Indolyl oder Phenyl, welches seinerseits gegebenenfalls mit Halogen, Nitro, $C_1$-$C_4$-Alkoxy oder $C_1$-$C_4$-Alkyl 1-, 2- oder 3-fach substituiert ist, wobei die genannten $C_3$- und $C_4$-Alkylreste auch verzweigt sein können und wobei bei Mehrfachsubstitutionen die Substituenten auch voneinander unabhängig verschieden sein können

oder $R^{1'}$ gesättigtes $C_5$-$C_7$-Cycloalkyl, welches gegebenenfalls benzoanneliert ist

oder $R^{1'}$ Phenyl, Naphthyl oder ein 5- oder 6-gliedriger aromatischer Ring mit 1, 2 oder 3 Stickstoff- und/oder Sauerstoff- und/oder Schwefelatomen, jeweils gegebenfalls mit Halogen, Nitro, Cyano, $C_1$-$C_4$-Alkyl oder $C_1$-$C_4$-Alkoxy 1-, 2- oder 3-fach substituiert, wobei bei Mehrfachsubstitutionen die Substituenten auch voneinander unabhängig verschieden sein können und wobei im Falle der $C_3$- und $C_4$-Alkyle diese auch verzweigt sein können,

$R^{2'}$ unabhängig von $R^{1'}$ Wasserstoff ist oder eine der Bedeutungen wie für $R^{1'}$ beschrieben hat, wobei $R^{2'}$ auch identisch mit $R^{1'}$ sein kann und

$R^{3'}$ unabhängig von $R^{1'}$ Wasserstoff ist oder eine der Bedeutungen wie für $R^{1'}$ beschrieben hat,

wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat wobei $R^{4'}$ auch identisch mit $R^{1'}$ und/oder $R^{2'}$ und/oder $R^{3'}$ sein kann

und wobei die Reste $R^{1'}$ und $R^{2'}$ und/oder $R^{3'}$ und $R^{4'}$ auch zusammen mit dem Stickstoffatom einen 5-, 6-oder 7-gliedrigen gesättigten heterocyclischen Ring bilden können, wobei der heterocyclische Ring auch ein zweites Stickstoffatom beinhalten kann und wobei der heterocyclische Ring seinerseits mit Phenyl oder Phenyl-$C_1$-$C_3$-alkyl substituiert sein kann,

bedeutet,

sowie deren physiologisch verträgliche Salze, ausgenommen die Verbindungen, in denen $R^{1'}$ = $R^{3'}$ 2-Hydroxyethyl oder 2-(3,4-dimethoxyphenyl)ethyl oder mit Methyl und Brom disubstituiertes Phenyl und $R^{2'}$ = $R^{4'}$ Wassertoff bedeutet und N,N,N',N'-Tetracyclohexyl-2,5-pyridindicarbonsäurediamid.

**4.** Pyridin-2,4- und -2,5-dicarbonsäure-Amide gemäß Formel I' nach Anspruch 3, worin

$R^{1'}$ verzweigtes oder unverzweigtes $C_1$-$C_{12}$-Alkyl, welches gegebenenfalls einfach oder im Falle der $C_2$-$C_{12}$-Alkyleauch mehrfach substituiert ist mit Amino oder $C_1$-$C_3$-Alkoxy, wobei die Alkylreste 1-3 C-Atome aufweisen und im Falle der $C_3$-Alkylverbindungen auch verzweigt sein können oder

Indolyl oder Phenyl, welches substituiert sein kann mit $C_1$-$C_4$-Alkoxy oder Nitro

oder $R^{1'}$ benzoanneliertes Cyclohexyl

oder $R^{1'}$ Phenyl, welches gegebenenfalls mit 1, 2 oder 3 Cyano-, Halogen-, $C_1$-$C_4$-Alkyl- oder Nitrogruppen substituiert ist und wobei bei Mehrfachsubstitutionen die Substituenten auch unabhängig voneinander verschieden sein können

oder $R^{1'}$ 2-, 3- oder 4-Pyridyl, 3-Thienyl oder Thiazolyl,

und

$R^{2'}$ unabhängig von $R^{1'}$ Wasserstoff ist oder eine der Bedeutungen wie für $R^{1'}$ beschrieben hat, wobei $R^{2'}$ auch identisch mit $R^{1'}$ sein kann und

$R^{3'}$ unabhängig von $R^{1'}$ und $R^{2'}$ Wasserstoff ist oder eine der Bedeutungen wie für $R^{1'}$ beschrieben hat, wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann

bedeutet,

sowie deren physiologisch verträgliche Salze, ausgenommen die Verbindungen, in denen $R^{1'}$ = $R^{3'}$ 2-Hydroxyethyl oder 2-(3,4-Dimethoxyphenyl)ethyl oder mit Methyl und Brom disubstituiertes Phenyl und $R^{2'}$ = $R^{4'}$ Wasserstoff bedeutet.

**5.** Verfahren zur Herstellung von Verbindungen der Formel I' nach Anspruch 3, dadurch gekennzeichnet, daß man

eine Verbindung der Formel II

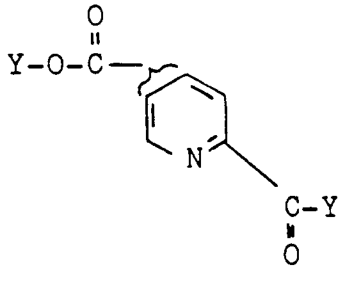

(II)

mit einer Verbindung der Formel III

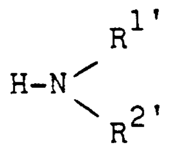

(III)

umsetzt, wobei $R^{1'} = R^{3'}$, $R^{2'} = R^{4'}$ die zu Formel I' angegebenen Bedeutungen haben und Y Halogen oder Hydroxy ist oder zusammen mit der Carbonylgruppe einen Aktivester oder ein gemischtes Anhydrid bildet und die Reaktionsprodukte gegebenenfalls in ihre physiologisch verträglichen Salze überführt.

**6.** Verfahren zur Herstellung von Verbindungen der Formel I' nach Anspruch 3, dadurch gekennzeichnet, daß man
eine Verbindung der Formel II

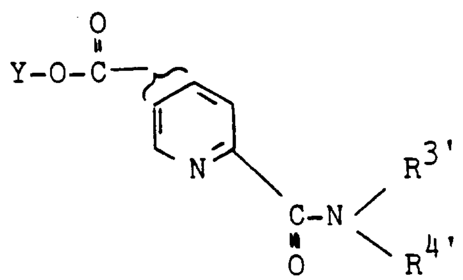

(II')

mit einer Verbindung der Formel III

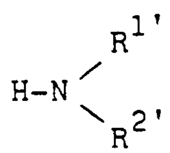

(III)

umsetzt, wobei $R^{1'}$, $R^{2'}$, $R^{3'}$ und $R^{4'}$ die zu Formel I' angegebenen Bedeutungen haben und Y Halogen oder Hydroxy ist oder zusammen mit der Carbonylgruppe einen Aktivester oder ein gemischtes Anhydrid bildet und die Reaktionsprodukte gegebenenfalls in ihre physiologisch verträglichen Salze überführt.

**7.** Verfahren nach Anspruch 5, dadurch gekennzeichnet, daß die Umsetzung unter gleichzeitiger Hinzugabe von Dialkylcarbodiimid erfolgt, wobei die Dialkylreste 1 bis 8 C-Atome aufweisen die im Falle der $C_3$-$C_8$-Verbindungen auch verzweigt oder cyclisch sein können.

**8.** Verbindungen nach einem der Ansprüche 1 bis 4 zur Inhibierung der Prolin- und Lysinhydroxylase.

**9.** Verbindungen nach Anspruch 1 bis 4 zur Anwendung als Fibrosuppressiva und Immunsuppressiva.

**10.** Arzneimittel, enthaltend eine Verbindung der Formel I oder I' gemäß Anspruch 1 oder 3 mit verträglichen pharmazeutischen Trägern.

**11.** Verfahren zur Herstellung von Arzneimitteln zur Beeinflussung des Stoffwechsels von Collagen und collagenähnlichen Stoffen bzw. der Biosynthese von $C1_q$, dadurch gekennzeichnet, daß man eine Verbindung der Formel I oder I' gemäß Anspruch 1 oder 3 in eine geeignete Darreichungsform bringt.

12. Pyrdin-2,4- und -2,5-dicarbonsäure-Amide gemäß Formel I' nach Anspruch 3, worin
    - $R^{1'}$ verzweigtes oder unverzweigtes $C_1$-$C_4$-Alkyl, welches substituiert ist mit $C_1$-$C_3$-Alkoxy, wobei die Alkylreste 1 - 3 C-Atome aufweisen und im Falle der $C_3$-Alkylverbindungen auch verzweigt sein können, und
    - $R^{2'}$ unabhängig von $R^{1'}$ Wasserstoff ist oder eine der Bedeutungen wie für $R^{1'}$ beschrieben hat, wobei $R^{2'}$ auch identisch mit $R^{1'}$ sein kann und
    - $R^{3'}$ unabhängig von $R^{1'}$ Wasserstoff ist oder eine der Bedeutungen wie für $R^{1'}$ beschrieben hat, wobei $R^{3'}$ auch identisch mit $R^{1'}$ und/oder $R^{2'}$ und/oder $R^{3'}$ sein kann und
    - $R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann

    sowie deren physiologisch verträgliche Salze.

13. Pyridin-2,4- und -2,5-dicarbonsäureamide gemäß Formel I' nach Anspruch 3, worin $R^{1'}$ und $R^{3'}$ eine 3-Isopropoxypropyl-Gruppe ist und $R^{2'}$ und $R^{4'}$ Wasserstoff ist, sowie deren physiologisch verträgliche Salze.

14. Pyridin-2,4- und -2,5-dicarbonsäureamide gemäß Formel I' nach Anspruch 3, worin $R^{1'}$ und $R^{3'}$ eine 2-Methoxyethyl-Gruppe ist und $R^{2'}$ und $R^{4'}$ Wasserstoff ist, sowie deren physiologisch verträgliche Salze.

**Patentansprüche für folgende Vertragsstaaten : ES, GR**

1. Verfahren zur Herstellung von Arzneimitteln, dadurch gekennzeichnet, daß man Pyridin-2,4- und -2,5-dicarbonsäure-Amide der Formel I

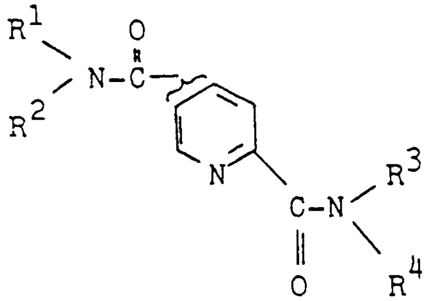

(I)

worin

- $R^1$ verzweigtes oder unverzweigtes $C_1$-$C_{12}$-Alkyl, welches gegebenenfalls einfach oder im Falle der $C_2$-$C_{12}$-Alkyleauch mehrfach substituiert ist mit

  Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, die im Falle der $C_3$- und $C_4$-Alkylreste auch verzweigt sein können,

  Indolyl oder Phenyl, welches seinerseits gegebenenfalls mit Halogen, Nitro, $C_1$-$C_4$-Alkyl oder $C_1$-$C_4$-Alkoxy, 1-, 2-, oder 3-fach substituiert ist, wobei bei Mehrfachsubstitutionen die Substituenten auch voneinander unabhängig verschieden sein können und wobei im Falle der $C_3$- und $C_4$-Alkyle diese auch verzweigt sein können,

  oder $R^1$ gesättigtes $C_5$-$C_7$-Cycloalkyl, welches gegebenenfalls benzoanneliert ist,

  oder $R^{1'}$ Phenyl, Naphthyl oder ein 5- oder 6-gliedriger aromatischer Ring mit 1, 2 oder 3 Stickstoff- und/oder Sauerstoff- und/oder Schwefelatomen, jeweils gegebenenfalis mit Halogen, Nitro, Cyano, $C_1$-$C_4$-Alkyl oder $C_1$-$C_4$-Alkoxy 1-, 2-, oder 3-fach substituiert, wobei bei Mehrfachsubstitutionen die Substituenten auch voneinander unabhängig verschieden sein können und wobei im Falle der $C_3$- und $C_4$-Alkyle diese auch verzweigt sein können

und

- $R^2$ unabhängig von $R^1$ Wasserstoff ist oder eine der Bedeutungen wie für $R^1$ beschrieben hat, wobei $R^2$ auch identisch mit $R^1$ sein kann und
- $R^3$ unabhängig von $R^1$ und $R^2$ Wasserstoff ist oder eine der Bedeutungen wie für $R^1$ beschrieben hat, wobei $R^3$ auch identisch mit $R^1$ und/oder $R^2$ sein kann und
- $R^4$ unabhängig von $R^1$ $R^2$ und $R^3$ Wasserstoff ist oder eine der Bedeutungen wie für $R^1$

beschrieben hat, wobei $R^4$ auch identisch mit $R^1$ und/oder $R^2$ und/oder $R^3$ sein kann

und wobei die Reste $R^1$ und $R^2$ und/oder $R^3$ und $R^4$ auch zusammen mit dem Stickstoffatom einen 5-, 6- oder 7-gliedrigen gesättigten heterocyclischen Ring bilden können, wobei der heterocyclische Ring auch ein zweites Stickstoffatom beinhalten kann und wobei der heterocyclische Ring seinerseits mit Phenyl oder Phenyl-$C_1$-$C_3$-Alkyl substituiert sein kann,

bedeutet und deren physiologisch verträgliche Salze, ausgenommen die Verbindungen, in denen $R^1 = R^3$ mit Methyl und Brom disubstituiertes Phenyl und $R^2 = R^4$ Wasserstoff bedeutet, in eine geeignete Darreichungsform bringt.

**2.** Verfahren zur Herstellung von Arzneimitteln, dadurch gekennzeichnet, daß man Pyridin-2,4- und -2,5-dicarbonsäure-Amide nach Anspruch 1 gemäß Formel I, worin

$R^1$ verzweigtes oder unverzweigtes $C_1$-$C_{12}$-Alkyl welches gegebenenfalls einfach oder im Falle der $C_3$-$C_{12}$-Alkyleauch mehrfach substituiert ist, mit Amino oder $C_1$-$C_3$-Alkoxy wobei die $C_3$-AlkylReste auch verzweigt sein können und/oder Indolyl und/oder Phenyl, welches substituiert sein kann mit $C_1$-$C_4$-Alkoxy oder Nitro

oder $R^1$ benzoanneliertes Cyclohexyl

oder $R^1$ Phenyl, das gegebenenfalls 1-, 2- oder 3-fach Nitro-Cyano-, Halogen- oder $C_1$-$C_4$-Alkyl substituiert ist, wobei bei Mehrfachsubstitutionen die Substituenten auch unabhängig voneinander verschieden sein können

oder $R^1$ 2-, 3- oder 4-Pyridyl, 2- oder 3-Thienyl oder Thiazolyl wobei diese Heteroaromaten auch mit $C_1$-$C_4$-Alkyl monosubstituiert sein können

bedeutet und

$R^2$ unabhängig von $R^1$ Wasserstoff ist oder eine der Bedeutungen wie für $R^1$ beschrieben hat, wobei $R^2$ auch identisch mit $R^1$ sein kann und

$R^3$ unabhängig von $R^1$ und $R^2$ Wasserstoff ist oder eine der Bedeutungen wie für $R^1$ beschrieben hat, wobei $R^3$ auch identisch mit $R^1$ und/oder $R^2$ sein kann und

$R^4$ unabhängig von $R^1$, $R^2$ und $R^3$ Wasserstoff ist oder eine der Bedeutungen wie für $R^1$ beschrieben hat, wobei $R^4$ auch identisch mit $R^1$ und/oder $R^2$ und/oder $R^3$ sein kann

bedeutet, sowie deren physiologisch verträgliche Salze, ausgenommen die Verbindungen, in denen $R^1 = R^3$ mit Methyl und Brom disubstituiertes Phenyl und $R^2 = R^4$ Wasserstoff bedeutet, in eine geeignete Darreichungsform bringt.

**3.** Verfahren zur Herstellung von Pyridin-2,4- und -2,5-dicarbonsäure-Amide der Formel I',

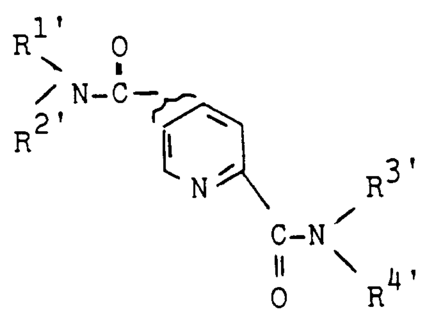

(I')

worin

$R^{1'}$ verzweigtes oder unverzweigtes $C_1$-$C_{12}$-Alkyl, welches gegebenenfalls einfach oder im Falle von $C_2$-$C_{12}$-Alkyl auch mehrfach substituiert ist mit Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, und wobei im Falle der $C_3$- und $C_4$Alkyle diese auch verzweigt sein können, Indolyl oder Phenyl, welches seinerseits gegebenenfalls mit Halogen, Nitro, $C_1$-$C_4$-Alkoxy oder $C_1$-$C_4$-Alkyl 1-, 2- oder 3-fach substituiert ist, wobei die genannten $C_3$- und $C_4$-Alkylreste auch verzweigt sein können und wobei bei Mehrfachsubstitutionen die Substituenten auch voneinander unabhängig verschieden sein können

oder $R^{1'}$ gesättigtes $C_5$-$C_7$-Cycloalkyl, welches gegebenenfalls benzoanneliert ist

oder $R^{1'}$ Phenyl, Naphthyl oder ein 5- oder 6-gliedriger aromatischer Rind mit 1, 2 oder 3

Stickstoff- und/oder Sauerstoff- und/oder Schwefelatomen, jeweils gegebenenfalls mit Halogen, Nitro, Cyano, $C_1$-$C_4$-Alkyl oder $C_1$-$C_4$-Alkoxy 1-, 2- oder 3-fach substituiert ist, wobei bei Mehrfachsubstitutionen die Substituenten auch voneinander unabhängig verschieden sein können und wobei im Falle der $C_3$- und $C_4$-Alkyle diese auch verzweigt sein können,

$R^{2'}$ unabhängig von $R^{1'}$ Wasserstoff ist oder eine der Bedeutungen wie für $R^{1'}$ beschrieben hat, wobei $R^{2'}$ auch identisch mit $R^{1'}$ sein kann und

$R^{3'}$ unabhängig von $R^{1'}$ Wasserstoff ist oder eine der Bedeutungen wie für $R^{1'}$ beschrieben, hat, wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat wobei $R^{4'}$ auch identisch mit $R^{1'}$ und/oder $R^{2'}$ und/oder $R^{3'}$ sein kann

und wobei die Reste $R^{1'}$ und $R^{2'}$ und/oder $R^{3'}$ und $R^{4'}$ auch zusammen mit dem Stickstoffatom einen 5-, 6-oder 7-gliedrigen gesättigten heterocyclischen Ring bilden können, wobei der heterocyclische Ring auch ein zweites Stickstoffatom beinhalten kann und wobei der heterocyclische Ring seinerseits mit Phenyl oder Phenyl-$C_1$-$C_3$-alkyl substituiert sein kann,

bedeutet,

sowie deren physiologisch verträglichen Salzen, ausgenommen die Verbindungen, in denen $R^{1'}$ = $R^{3'}$ 2-Hydroxyethyl oder 2-(3,4-dimethoxyphenyl)ethyl oder mit Methyl und Brom disubstituiertes Phenyl und $R^{2'}$ = $R^{4'}$ Wasserstoff bedeutet und N,N,N',N'-Tetracyclohexyl-2,5-pyridindicarbonsäurediamid, dadurch gekennzeichnet, daß man

eine Verbindung der Formel II

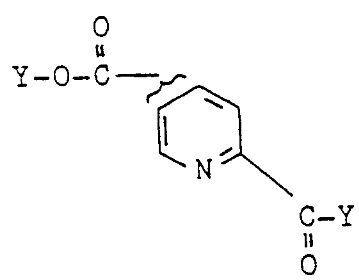

(II)

mit einer Verbindung der Formel III

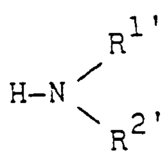

(III)

umsetzt, wobei $R^{1'}$ = $R^{3'}$, $R^{2'}$ = $R^{4'}$ die zu Formel I' angegebenen Bedeutungen haben und Y Halogen oder Hydroxy ist oder zusammen mit der Carbonylgruppe einen Aktivester oder ein gemischtes Anhydrid bildet und die Reaktionsprodukte gegebenenfalls in ihre physiologisch verträglichen Salze überführt.

4. Verfahren zur Herstellung von Verbindungen der Formel I' nach Anspruch 3, dadurch gekennzeichnet, daß man

eine Verbindung der Formel II

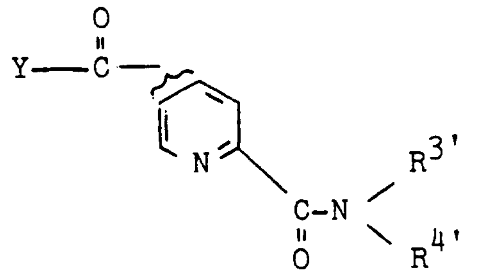

(II')

mit einer Verbindung der Formel III

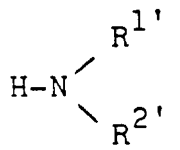

(III)

umsetzt, wobei $R^{1'}$, $R^{2'}$, $R^{3'}$ und $R^{4'}$ die zu Formel I' angegebenen Bedeutungen haben und Y Halogen oder Hydroxy ist oder zusammen mit der Carbonylgruppe einen Aktivester oder ein gemischtes Anhydrid bildet und die Reaktionsprodukte gegebenenfalls in ihre physiologisch verträglichen Salze überführt.

**5.** Verfahren nach Anspruch 3 oder 4 zur Herstellung von Pyridin-2,4- und -2,5-dicarbonsäure-Amiden gemäß Formel I' worin

$R^{1'}$ verzweigtes oder unverzweigtes $C_1$-$C_{12}$-Alkyl, welches gegebenenfalls einfach oder im Falle der $C_2$-$C_{12}$-Alkyleauch mehrfach substituiert ist mit Amino oder $C_1$-$C_3$-Alkoxy, wobei die Alkylreste 1-3 C-Atome aufweisen und im Falle der $C_3$-Alkylverbindungen auch verzweigt sein können oder Indolyl oder Phenyl, welches substituiert sein kann mit $C_1$-$C_4$-Alkoxy oder Nitro

oder $R^{1'}$ benzoanneliertes Cyclohexyl

oder $R^{1'}$ Phenyl, welches gegebenenfalls mit 1, 2 oder 3 Cyano-, Halogen-, $C_1$-$C_4$-Alkyl- oder Nitrogruppen substituiert ist und wobei bei Mehrfachsubstitutionen die Substituenten auch unabhängig voneinander verschieden sein können

oder $R^{1'}$ 2-, 3- oder 4-Pyridyl, 3-Thienyl oder Thiazolyl,

und

$R^{2'}$ unabhängig von $R^{1'}$, Wasserstoff ist oder eine der Bedeutungen wie für $R^{1'}$ beschrieben hat, wobei $R^{2'}$ auch identisch mit $R^{1'}$ sein kann und

$R^{3'}$ unabhängig von $R^{1'}$ und $R^{2'}$ Wasserstoff ist oder eine der Bedeutungen wie für $R^{1'}$ beschrieben hat, wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann

bedeutet,

sowie deren physiologisch verträglichen Salzen, ausgenommen die Verbindungen, in denen $R^{1'}$ = $R^{3'}$ 2-Hydroxyethyl oder 2-(3,4-dimethoxyphenyl)ethyl oder mit Methyl und Brom disubstituiertes Phenyl und $R^{2'}$ = $R^{4'}$ Wasserstoff bedeutet.

**6.** Verfahren nach Anspruch 3 oder 4, dadurch gekennzeichnet, daß die Umsetzung unter gleichzeitiger Hinzugabe von Dialkylcarbodimid erfolgt, wobei die Dialkylreste 1 bis 8 C-Atome aufweisen, die im Falle der $C_3$-$C_8$-Verbindungen auch verzweigt oder cyclisch sein können.

**7.** Verfahren zur Herstellung von Arzneimitteln zur Inhibierung der Prolin- und Lysinhydroxylase, dadurch gekennzeichnet, daß man eine Verbindung der Formel I oder I' gemäß Anspruch 1 oder 3 in eine geeignete Darreichungsform bringt.

**8.** Verfahren zur Herstellung von Arzneimitteln zur Anwendung als Fibrosuppressiva und Immunsuppressiva, dadurch gekennzeichnet, daß man eine Verbindung der Formel I oder I' gemäß Anspruch 1 oder 3 in eine geeignete Darreichungsform bringt.

**9.** Verfahren zur Herstellung von Arzneimitteln zur Beeinflussung des Stoffwechsels von Collagen und collagenähnlichen Stoffen bzw. der Biosynthese von $C1_q$, dadurch gekennzeichnet, daß man eine Verbindung der Formel I oder I' gemäß Anspruch 1 oder 3 in eine geeignete Darreichungsform bringt.

**10.** Verfahren nach Anspruch 3 oder 4 zur Herstellung von Pyridin-2,4- und -2,5-dicarbonsäure-Amiden gemäß Formel I' nach Anspruch 3, worin

$R^{1'}$ verzweigtes oder unverzweigtes $C_1$-$C_4$-Alkyl, welches substituiert ist mit $C_1$-$C_3$-Alkoxy, wobei die Alkylreste 1 - 3 C-Atome aufweisen und im Falle der $C_3$-Alkylverbindungen auch verzweigt sein können, und

$R^{2'}$ unabhängig von $R^{1'}$ Wasserstoff ist oder eine der Bedeutungen wie für $R^{1'}$ beschrieben hat, wobei $R^{2'}$ auch identisch mit $R^{1'}$ sein kann und

$R^{3'}$ unabhängig von $R^{1'}$ Wasserstoff ist oder eine der Bedeutungen wie für $R^{1'}$ beschrieben hat, wobei $R^{3'}$ auch identisch mit $R^{1'}$ und/oder $R^{2'}$ sein kann und

$R^{4'}$ unabhängig von $R^{1'}$, $R^{2'}$ und $R^{3'}$ Wasserstoff ist oder eine der Bedeutungen wie für $R^{1'}$ beschrieben hat, wobei $R^{4'}$ auch identisch mit $R^{1'}$ und/oder $R^{2'}$ und/oder $R^{3'}$ sein kann.

sowie deren physiologisch verträglichen Salzen.

**11.** Verfahren nach Anspruch 3 oder 4 zur Herstellung von Pyridin-2,4- und -2,5-dicarbonsäureamiden gemäß Formel I' nach Anspruch 3, worin $R^{1'}$ und $R^{3'}$ eine 3-Isopropoxypropyl-Gruppe ist und $R^{2'}$ und $R^{4'}$ Wasserstoff ist, sowie deren physiologisch verträglichen Salzen.

**12.** Verfahren nach Anspruch 3 oder 4 zur Herstellung von Pyridin-2,4- und -2,5-dicarbonsäureamiden gemäß Formel I' nach Anspruch 3, worin $R^{1'}$ und $R^{3'}$ eine 2-Methoxyethyl-Gruppe ist und $R^{2'}$ und $R^{4'}$ Wasserstoff ist, sowie deren physiologisch verträglichen Salzen.

## Claims

**Claims for the following Contracting States : AT, BE, CH, DE, FR, GB, IT, LI, LU, NL, SE**

**1.** A pyridine-2,4- or -2,5-dicarboxylic acid amide of the formula I

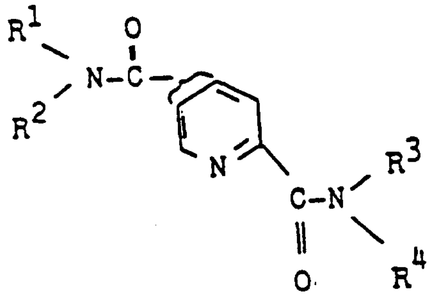

(I)

in which

$R^1$ denotes branched or unbranched $C_1$-$C_{12}$-alkyl which is optionally monosubstituted or, in the case of the $C_2$-$C_{12}$-alkyl radicals, also polysubstituted by halogen, hydroxyl, cyano, amino, carboxyl, alkoxy, alkoxycarbonyl, alkylcarbonyloxy or alkyl- or dialkylamino, where the alkyl radicals contain 1-4 carbon atoms which, in the case of the $C_3$- and $C_4$-alkyl radicals, can also be branched, indolyl or phenyl, which is in turn optionally mono-, di- or trisubstituted by halogen, nitro, $C_1$-$C_4$-alkyl or $C_1$-$C_4$-alkoxy, it also being possible, in the case of polysubstitution, for the substituents to be different independently of one another and, in the case of the $C_3$- and $C_4$-alkyl radicals, it also being possible for these to be branched,

or $R^1$ denotes saturated $C_5$-$C_7$-cycloalkyl, which is optionally benzo-fused,

$R^1$ denotes phenyl, naphthyl or a 5- or 6-membered aromatic ring having 1, 2 or 3 nitrogen

and/or oxygen and/or sulfur atoms, in each case optionally mono-, di- or trisubstituted by halogen, nitro, cyano, $C_1$-$C_4$-alkyl or $C_1$-$C_4$-alkoxy, it also being possible, in the case of polysubstitution, for the substituents to be different independently of one another and, in the case of the $C_3$- and $C_4$-alkyl radicals, it also being possible for these to be branched,

and

$R^2$, independently of $R^1$, is hydrogen or has one of the meanings described for $R^1$, it also being possible for $R^2$ to be identical to $R^1$, and

$R^3$, independently of $R^1$ and $R^2$, is hydrogen or has one of the meanings described for $R^1$, it also being possible for $R^3$ to be identical to $R^1$ and/or $R^2$, and

$R^4$, independently of $R^1$, $R^2$ and $R^3$, is hydrogen or has one of the meanings described for $R^1$, it also being possible for $R^4$ to be identical to $R^1$ and/or $R^2$ and/or $R^3$,

and in which the radicals $R^1$ and $R^2$ and/or $R^3$ and $R^4$, together with the nitrogen atom, can also form a 5-, 6- or 7-membered saturated heterocyclic ring, it also being possible for the heterocyclic ring to include a second nitrogen atom and it being possible for the heterocyclic ring in turn to be substituted by phenyl or phenyl-$C_1$-$C_3$-alkyl,

or a physiologically tolerated salt thereof, for use as a medicament, excluding the compounds in which $R^1 = R^3$ denotes phenyl which is disubstituted by methyl and bromine and $R^2 = R^4$ denotes hydrogen.

**2.** A pyridine-2,4- or -2,5-dicarboxylic acid amide as claimed in claim 1 according to formula I, in which

$R^1$ denotes branched or unbranched $C_1$-$C_{12}$-alkyl which is optionally monosubstituted or, in the case of the $C_3$-$C_{12}$-alkyl radicals, also polysubstituted by amino or $C_1$-$C_3$-alkoxy, it also being possible for the $C_3$-alkyl radicals to be branched, and/or indolyl and/or phenyl, which can be substituted by $C_1$-$C_4$-alkoxy or nitro,

or $R^1$ denotes benzo-fused cyclohexyl,

or $R^1$ denotes phenyl, which is optionally mono-, di- or trisubstituted by nitro, cyano, halogen or $C_1$-$C_4$-alkyl, it also being possible, in the case of polysubstitution, for the substituents to be different independently of one another,

or $R^1$ denotes 2-, 3- or 4-pyridyl, 2- or 3-thienyl or thiazolyl, it also being possible for these heteroaromatics to be monosubstituted by $C_1$-$C_4$-alkyl,

and

$R^2$, independently of $R^1$, is hydrogen or has one of the meanings described for $R^1$, it also being possible for $R^2$ to be identical to $R^1$, and

$R^3$, independently of $R^1$ and $R^2$, is hydrogen or has one of the meanings described for $R^1$, it also being possible for $R^3$ to be identical to $R^1$ and/or $R^2$, and

$R^4$, independently of $R^1$, $R^2$ and $R^3$, is hydrogen or has one of the meanings described for $R^1$, it also being possible for $R^4$ to be identical to $R^1$ and/or $R^2$ and/or $R^3$,

or a physiologically tolerated salt thereof, for use as a medicament, excluding the compounds in which $R^1 = R^3$ denotes phenyl which is disubstituted by methyl and bromine and $R^2 = R^4$ denotes hydrogen.

**3.** A pyridine-2,4- or -2,5-dicarboxylic acid amide of the formula I'

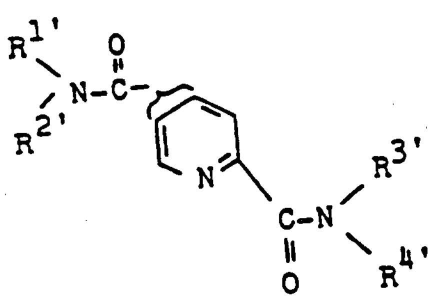

(I')

in which

$R^{1'}$ denotes branched or unbranched $C_1$-$C_{12}$-alkyl which is optionally monosubstituted or, in the case of $C_2$-$C_{12}$-alkyl, also polysubstituted by halogen, hydroxyl, cyano, amino, carboxyl, alkoxy, alkoxycarbonyl, alkylcarbonyloxy, alkyl- or dialkylamino, where the alkyl radicals contain 1-4 carbon atoms, and it also being possible, in the case of the $C_3$- and $C_4$-alkyl radicals, for these to be branched, indolyl or phenyl, which is in turn optionally mono-, di- or

trisubstituted by halogen, nitro, $C_1$-$C_4$-alkoxy or $C_1$-$C_4$-alkyl, it also being possible for the $C_3$- and $C_4$-alkyl radicals mentioned to be branched and it also being possible, in the case of polysubstitution, for the substituents to be different independently of one another,

or $R^{1'}$ denotes saturated $C_5$-$C_7$-cycloalkyl, which is optionally benzo-fused,

or $R^{1'}$ denotes phenyl, naphthyl or a 5- or 6-membered aromatic ring having 1, 2 or 3 nitrogen and/or oxygen and/or sulfur atoms, in each case optionally mono-, di- or trisubstituted by halogen, nitro, cyano, $C_1$-$C_4$-alkyl or $C_1$-$C_4$-alkoxy, it also being possible, in the case of polysubstitution, for the substituents to be different independently of one another and it also being possible, in the case of the $C_3$- and $C_4$-alkyl radicals, for these to be branched,

and

$R^{2'}$, independently of $R^{1'}$, is hydrogen or has one of the meanings described for $R^{1'}$, it also being possible for $R^{2'}$ to be identical to $R^{1'}$, and

$R^{3'}$, independently of $R^{1'}$, is hydrogen or has one of the meanings described for $R^{1'}$, it also being possible for $R^{3'}$ to be identical to $R^{1'}$ and/or $R^{2'}$, and

$R^{4'}$, independently of $R^{1'}$, $R^{2'}$ and $R^{3'}$, is hydrogen or has one of the meanings described for $R^{1'}$, it also being possible for $R^{4'}$ to be identical to $R^{1'}$ and/or $R^{2'}$ and/or $R^{3'}$,

and in which the radicals $R^{1'}$ and $R^{2'}$ and/or $R^{3'}$ and $R^{4'}$, together with the nitrogen atom, can also form a 5-, 6- or 7-membered saturated heterocyclic ring, it also being possible for the heterocyclic ring to include a second nitrogen atom and it being possible for the heterocyclic ring in turn to be substituted by phenyl or phenyl-$C_1$-$C_3$-alkyl,

or a physiologically tolerated salt thereof, excluding the compounds in which $R^{1'} = R^{3'}$ denotes 2-hydroxyethyl or 2-(3,4-dimethoxyphenyl)ethyl or phenyl which is disubstituted by methyl and bromine and $R^{2'} = R^{4'}$ denotes hydrogen, and N,N,N',N'-tetracyclohexyl-2,5-pyridinedicarboxylic acid diamide.

**4.** A pyridine-2,4- or -2,5-dicarboxylic acid amide according to formula I' as claimed in claim 3, in which

$R^{1'}$ denotes branched or unbranched $C_1$-$C_{12}$-alkyl which is optionally monosubstituted or, in the case of the $C_2$-$C_{12}$-alkyl radicals, also polysubstituted by amino or $C_1$-$C_3$-alkoxy, where the alkyl radicals contain 1-3 carbon atoms, and it also being possible, in the case of the $C_3$-alkyl compounds, for the alkyl radicals to be branched, or indolyl or phenyl, which can be substituted by $C_1$-$C_4$-alkoxy or nitro,

or $R^{1'}$ denotes benzo-fused cyclohexyl,

or $R^{1'}$ denotes phenyl, which is optionally substituted by 1, 2 or 3 cyano, halogen, $C_1$-$C_4$-alkyl or nitro groups, it also being possible, in the case of polysubstitution, for the substituents to be different independently of one another,

or $R^{1'}$ denotes 2-, 3- or 4-pyridyl, 3-thienyl or thiazolyl, and

$R^{2'}$, independently of $R^{1'}$, is hydrogen or has one of the meanings described for $R^{1'}$, it also being possible for $R^{2'}$ to be identical to $R^{1'}$, and

$R^{3'}$, independently of $R^{1'}$ and $R^{2'}$, is hydrogen or has one of the meanings described for $R^{1'}$, it also being possible for $R^{3'}$ to be identical to $R^{1'}$ and/or $R^{2'}$, and

$R^{4'}$, independently of $R^{1'}$, $R^{2'}$ and $R^{3'}$, is hydrogen or has one of the meanings described for $R^{1'}$, it also being possible for $R^{4'}$ to be identical to $R^{1'}$ and/or $R^{2'}$ and/or $R^{3'}$,

or a physiologically tolerated salt thereof, excluding the compounds in which $R^{1'}=R^{3'}$ denotes 2-hydroxyethyl or 2-(3,4-dimethoxyphenyl)ethyl or phenyl which is disubstituted by methyl and bromine and $R^{2'} = R^{4'}$ denotes hydrogen.

**5.** A process for the preparation of a compound of the formula I' as claimed in claim 3, which comprises reacting

a compound of the formula II

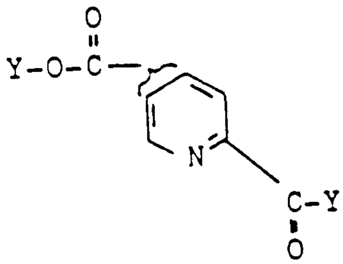

(II)

with a compound of the formula III

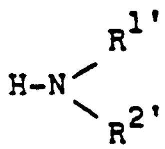

(III)

where $R^{1'} = R^{3'}$, $R^{2'} = R^{4'}$ have the meanings given for formula I', and Y is halogen or hydroxyl or, together with the carbonyl group, forms an active ester or a mixed anhydride, and, if appropriate, converting the reaction products into their physiologically tolerated salts.

6. A process for the preparation of a compound of the formula I' as claimed in claim 3, which comprises reacting
a compound of the formula II'

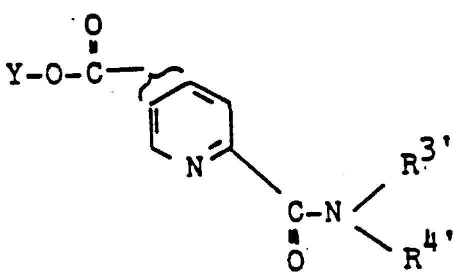

(II')

with a compound of the formula III

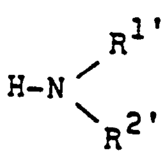

(III)

where $R^{1'}$, $R^{2'}$, $R^{3'}$ and $R^{4'}$ have the meanings given for formula I', and Y is halogen or hydroxyl or, together with the carbonyl group, forms an active ester or a mixed anhydride, and, if appropriate, converting the reaction products into their physiologically tolerated salts.

7. The process as claimed in claim 5, wherein the reaction is carried out with simultaneous addition of dialkylcarbodiimide, where the dialkyl radicals contain 1 to 8 carbon atoms which, in the case of the $C_3$-$C_8$-compounds, can also be branched or cyclic.

8. A compound as claimed in any of claims 1 to 4 for inhibiting proline hydroxylase and lysine hydroxylase.

**9.** A compound as claimed in any of claims 1 to 4 for use as fibrosuppressant and immunosuppressant.

**10.** A medicament containing a compound of the formula I or I' as claimed in claim 1 or 3 with tolerated pharmaceutical excipients.

**11.** A process for the preparation of a medicament for influencing the metabolism of collagen and collagen-like substances or the biosynthesis of $C1_q$, which comprises bringing a compound of the formula I or I' as claimed in claim 1 or 3 into a suitable dosage form.

**12.** A pyridine-2,4- or -2,5-dicarboxylic acid amide according to formula I' as claimed in claim 3, in which

$R^{1'}$ denotes branched or unbranched $C_1$-$C_4$-alkyl, which is substituted by $C_1$-$C_3$-alkoxy, it being possible for the alkyl radicals to contain 1-3 carbon atoms and, in the case of the $C_3$-alkyl compounds, also to be branched, and

$R^{2'}$, independently of $R^{1'}$, is hydrogen or has one of the meanings described for $R^{1'}$, it also being possible for $R^{2'}$ to be identical to $R^{1'}$, and

$R^{3'}$, independently of $R^{1'}$, is hydrogen or has one of the meanings described for $R^{1'}$, it also being possible for $R^{3'}$ to be identical to $R^{1'}$ and/or $R^{2'}$, and

$R^{4'}$, independently of $R^{1'}$, $R^{2'}$ and $R^{3'}$, is hydrogen or has one of the meanings described for $R^{1'}$, it also being possible for $R^{4'}$ to be identical to $R^{1'}$ and/or $R^{2'}$ and/or $R^{3'}$

or a physiologically tolerated salt thereof.

**13.** A pyridine-2,4- or -2,5-dicarboxylic acid amide according to formula I' as claimed in claim 3, in which $R^{1'}$ and $R^{3'}$ are a 3-isopropoxypropyl group and $R^{2'}$ and $R^{4'}$ are hydrogen, or a physiologically tolerated salt thereof.

**14.** A pyridine-2,4- or -2,5-dicarboxylic acid amide according to formula I' as claimed in claim 3, in which $R^{1'}$ and $R^{3'}$ are a 2-methoxyethyl group and $R^{2'}$ and $R^{4'}$ are hydrogen, or a physiologically tolerated salt thereof.

**Claims for the following Contracting States : ES, GR**

**1.** A process for the preparation of a medicament, which comprises bringing a pyridine-2,4- or -2,5-dicarboxylic acid amide of the formula I

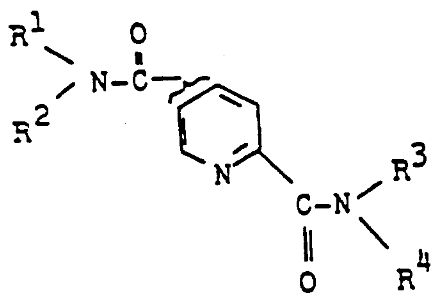

(I)

in which

$R^1$ denotes branched or unbranched $C_1$-$C_{12}$-alkyl which is optionally monosubstituted or, in the case of the $C_2$-$C_{12}$-alkyl radicals, also polysubstituted by halogen, hydroxyl, cyano, amino, carboxyl, alkoxy, alkoxycarbonyl, alkylcarbonyloxy or alkyl- or dialkylamino, where the alkyl radicals contain 1-4 carbon atoms which, in the case of the $C_3$- and $C_4$-alkyl radicals, can also be branched, indolyl or phenyl, which is in turn optionally mono-, di- or trisubstituted by halogen, nitro, $C_1$-$C_4$-alkyl or $C_1$-$C_4$-alkoxy, it also being possible, in the case of polysubstitution, for the substituents to be different independently of one another and, in the case of the $C_3$- and $C_4$-alkyl radicals, it also being possible for these to be branched,

or $R^1$ denotes saturated $C_5$-$C_7$-cycloalkyl, which is optionally benzo-fused,

or $R^1$ denotes phenyl, naphthyl or a 5- or 6-membered aromatic ring having 1, 2 or 3

nitrogen and/or oxygen and/or sulfur atoms, in each case optionally mono-, di- or trisubstituted by halogen, nitro, cyano, $C_1$-$C_4$-alkyl or $C_1$-$C_4$-alkoxy, it also being possible, in the case of polysubstitution, for the substituents to be different independently of one another and, in the case of the $C_3$- and $C_4$-alkyl radicals, it also being possible for these to be branched,

and

$R^2$, independently of $R^1$, is hydrogen or has one of the meanings described for $R^1$, it also being possible for $R^2$ to be identical to $R^1$, and

$R^3$, independently of $R^1$ and $R^2$, is hydrogen or has one of the meanings described for $R^1$, it also being possible for $R^3$ to be identical to $R^1$ and/or $R^2$, and

$R^4$, independently of $R^1$, $R^2$ and $R^3$, is hydrogen or has one of the meanings described for $R^1$, it also being possible for $R^4$ to be identical to $R^1$ and/or $R^2$ and/or $R^3$,

and in which the radicals $R^1$ and $R^2$ and/or $R^3$ and $R^4$, together with the nitrogen atom, can also form a 5-, 6- or 7-membered saturated heterocyclic ring, it also being possible for the heterocyclic ring to include a second nitrogen atom and it being possible for the heterocyclic ring in turn to be substituted by phenyl or phenyl-$C_1$-$C_3$-alkyl,

or a physiologically tolerated salt thereof, excluding the compounds in which $R^1 = R^3$ denotes phenyl which is disubstituted by methyl and bromine and $R^2 = R^4$ denotes hydrogen, into a suitable dosage form.

**2.** A process for the preparation of a medicament, which comprises bringing a pyridine-2,4- or -2,5-dicarboxylic acid amide as claimed in claim 1 according to formula I, in which

$R^1$ denotes branched or unbranched $C_1$-$C_{12}$-alkyl which is optionally monosubstituted or, in the case of the $C_3$-$C_{12}$-alkyl radicals, also polysubstituted by amino or $C_1$-$C_3$-alkoxy, it also being possible for the $C_3$-alkyl radicals to be branched, and/or indolyl and/or phenyl, which can be substituted by $C_1$-$C_4$-alkoxy or nitro,

or $R^1$ denotes benzo-fused cyclohexyl,

or $R^1$ denotes phenyl, which is optionally mono-, di- or trisubstituted by nitro, cyano, halogen or $C_1$-$C_4$-alkyl, it also being possible, in the case of polysubstitution, for the substituents to be different independently of one another,

or $R^1$ denotes 2-, 3- or 4-pyridyl, 2- or 3-thienyl or thiazolyl, it also being possible for these heteroaromatics to be monosubstituted by $C_1$-$C_4$-alkyl,

and

$R^2$, independently of $R^1$, is hydrogen or has one of the meanings described for $R^1$, it also being possible for $R^2$ to be identical to $R^1$, and

$R^3$, independently of $R^1$ and $R^2$, is hydrogen or has one of the meanings described for $R^1$, it also being possible for $R^3$ to be identical to $R^1$ and/or $R^2$, and

$R^4$, independently of $R^1$, $R^2$ and $R^3$, is hydrogen or has one of the meanings described for $R^1$, it also being possible for $R^4$ to be identical to $R^1$ and/or $R^2$ and/or $R^3$,

or a physiologically tolerated salt thereof, excluding the compounds in which $R^1 = R^3$ denotes phenyl which is disubstituted by methyl and bromine and $R^2 = R^4$ denotes hydrogen, into a suitable dosage form.

**3.** A process for the preparation of a pyridine-2,4- or -2,5-dicarboxylic acid amide of the formula I'

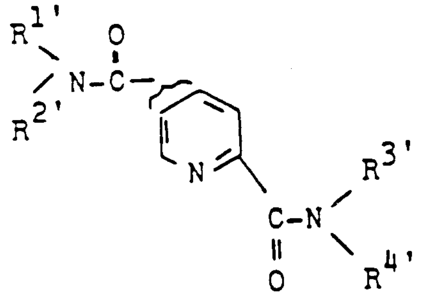

(I')

in which

$R^{1'}$ denotes branched or unbranched $C_1$-$C_{12}$-alkyl which is optionally monosubstituted or, in the

case of $C_2$-$C_{12}$-alkyl, also polysubstituted by halogen, hydroxyl, cyano, amino, carboxyl, alkoxy, alkoxy-carbonyl, alkylcarbonyloxy, alkyl- or dialkylamino, where the alkyl radicals contain 1-4 carbon atoms, and it also being possible, in the case of the $C_3$- and $C_4$-alkyl radicals, for these to be branched, indolyl or phenyl, which is in turn optionally mono-, di- or trisubstituted by halogen, nitro, $C_1$-$C_4$-alkoxy or $C_1$-$C_4$-alkyl, it also being possible for the $C_3$- and $C_4$-alkyl radicals mentioned to be branched and it also being possible, in the case of polysubstitution, for the substituents to be different independently of one another,

or $R^{1'}$ denotes saturated $C_5$-$C_7$-cycloalkyl, which is optionally benzo-fused,

or $R^{1'}$ denotes phenyl, naphthyl or a 5- or 6- membered aromatic ring having 1, 2 or 3 nitrogen and/or oxygen and/or sulfur atoms, in each case optionally mono-, di- or trisubstituted by halogen, nitro, cyano, $C_1$-$C_4$-alkyl or $C_1$-$C_4$-alkoxy, it also being possible, in the case of polysubstitution, for the substituents to be different independently of one another and it also being possible, in the case of the $C_3$- and $C_4$-alkyl radicals, for these to be branched,

and

$R^{2'}$, independently of $R^{1'}$, is hydrogen or has one of the meanings described for $R^{1'}$, it also the meanings described for $R^{1'}$, it also being possible for $R^{2'}$ to be identical to $R^{1'}$, and

$R^{3'}$, independently of $R^{1'}$, is hydrogen or has one of the meanings described for $R^{1'}$, it also being possible for $R^{3'}$ to be identical to $R^{1'}$ and/or $R^{2'}$, and

$R^{4'}$, independently of $R^{1'}$, $R^{2'}$ and $R^{3'}$, is hydrogen or has one of the meanings described for $R^{1'}$, it also being possible for $R^{4'}$ to be identical to $R^{1'}$ and/or $R^{2'}$ and/or $R^{3'}$,

and in which the radicals $R^{1'}$ and $R^{2'}$ and/or $R^{3'}$ and $R^{4'}$, together with the nitrogen atom, can also form a 5-, 6- or 7-membered saturated heterocyclic ring, it also being possible for the heterocyclic ring to include a second nitrogen atom and it being possible for the heterocyclic ring in turn to be substituted by phenyl or phenyl-$C_1$-$C_3$-alkyl,

or a physiologically tolerated salt thereof, excluding the compounds in which $R^{1'} = R^{3'}$ denotes 2-hydroxyethyl or 2-(3,4-dimethoxyphenyl)ethyl or phenyl which is disubstituted by methyl and bromine and $R^{2'} = R^{4'}$ denotes hydrogen, and N,N,N',N'-tetracyclohexyl-2,5-pyridinedicarboxylic acid diamide, which comprises reacting a compound of the formula II

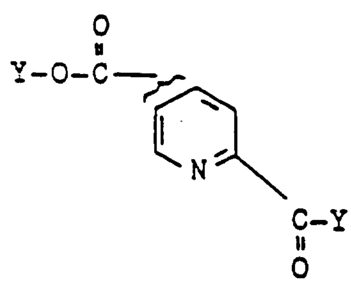

(II)

with a compound of the formula III

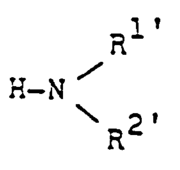

(III)

where $R^{1'} = R^{3'}$, $R^{2'} = R^{4'}$ have the meanings given for formula I', and Y is halogen or hydroxyl or, together with the carbonyl group, forms an active ester or a mixed anhydride, and, if appropriate, converting the reaction products into their physiologically tolerated salts.

4. The process for the preparation of a compound of the formula I' as claimed in claim 3, which comprises reacting
a compound of the formula II'

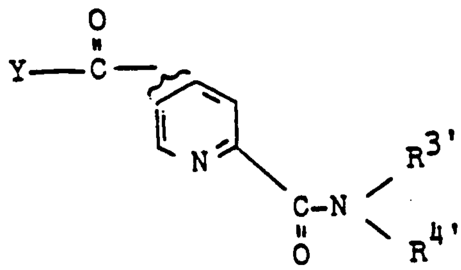

(II')

with a compound of the formula III

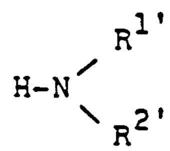

(III)

where $R^{1'}$, $R^{2'}$, $R^{3'}$ and $R^{4'}$ have the meanings given for formula I' and Y is halogen or hydroxyl or, together with the carbonyl group, forms an active ester or a mixed anhydride, and, if appropriate, converting the reaction products into their physiologically tolerated salts.

**5.** The process as claimed in claim 3 or 4 for the preparation of a pyridine-2,4- or -2,5-dicarboxylic acid amide according to formula I', in which

$R^{1'}$ denotes branched or unbranched $C_1$-$C_{12}$-alkyl which is optionally monosubstituted or, in the case of the $C_2$-$C_{12}$-alkyl radicals, also polysubstituted by amino or $C_1$-$C_3$-alkoxy, where the alkyl radicals contain 1-3 carbon atoms, and it also being possible, in the case of the $C_3$-alkyl compounds, for the alkyl radicals to be branched, or indolyl or phenyl, which can be substituted by $C_1$-$C_4$-alkoxy or nitro,

or $R^{1'}$ denotes benzo-fused cyclohexyl,

or $R^{1'}$ denotes phenyl, which is optionally substituted by 1, 2 or 3 cyano, halogen, $C_1$-$C_4$-alkyl or nitro groups, it also being possible, in the case of polysubstitution, for the substituents to be different independently of one another,

or $R^{1'}$ denotes 2-, 3- or 4-pyridyl, 3-thienyl or thiazolyl,

and

$R^{2'}$, independently of $R^{1'}$, is hydrogen or has one of the meanings described for $R^{1'}$, it also being possible for $R^{2'}$ to be identical to $R^{1'}$, and

$R^{3'}$, independently of $R^{1'}$ and $R^{2'}$, is hydrogen or has one of the meanings described for $R^{1'}$, it also being possible for $R^{3'}$ to be identical to $R^{1'}$ and/or $R^{2'}$, and

$R^{4'}$, independently of $R^{1'}$, $R^{2'}$ and $R^{3'}$, is hydrogen or has one of the meanings described for $R^{1'}$, it also being possible for $R^{4'}$ to be identical to $R^{1'}$ and/or $R^{2'}$ and/or $R^{3'}$,

or a physiologically tolerated salt thereof, excluding the compounds in which $R^{1'} = R^{3'}$ denotes 2-hydroxyethyl or 2-(3,4-dimethoxyphenyl)ethyl or phenyl which is disubstituted by methyl and bromine and $R^{2'} = R^{4'}$ denotes hydrogen.

**6.** The process as claimed in claim 3 or 4, wherein the reaction is carried out with simultaneous addition of dialkylcarbodiimide, where the dialkyl radicals contain 1 to 8 carbon atoms which, in the case of the $C_3$-$C_8$-compounds, can also be branched or cyclic.

**7.** A process for the preparation of a medicament for inhibiting proline hydroxylase and lysine hydroxylase, which comprises bringing a compound of the formula I or I' as claimed in claim 1 or 3 into a suitable dosage form.

**8.** A process for the preparation of a medicament for use as fibrosuppressant and immunosuppressant, which comprises bringing a compound of the formula I or I' as claimed in claim 1 or 3 into a suitable dosage form.

9. A process for the preparation of a medicament for influencing the metabolism of collagen and collagen-like substances or the biosynthesis of $C1_q$, which comprises bringing a compound of the formula I or I' as claimed in claim 1 or 3 into a suitable dosage form.

10. The process as claimed in claim 3 or 4 for the preparation of a pyridine-2,4- or -2,5-dicarboxylic acid amide according to formula I' as claimed in claim 3, in which
    - $R^{1'}$ denotes branched or unbranched $C_1$-$C_4$-alkyl, which is substituted by $C_1$-$C_3$-alkoxy, it being possible for the alkyl radicals to contain 1-3 carbon atoms and, in the case of the $C_3$-alkyl compounds, also to be branched, and
    - $R^{2'}$, independently of $R^{1'}$, is hydrogen or has one of the meanings described for $R^{1'}$, it also being possible for $R^{2'}$ to be identical to $R^{1'}$, and
    - $R^{3'}$, independently of $R^{1'}$, is hydrogen or has one of the meanings described for $R^{1'}$, it also being possible for $R^{3'}$ to be identical to $R^{1'}$ and/or $R^{2'}$, and
    - $R^{4'}$, independently of $R^{1'}$, $R^{2'}$ and $R^{3'}$, is hydrogen or has one of the meanings described for $R^{1'}$, it also being possible for $R^{4'}$ to be identical to $R^{1'}$ and/or $R^{2'}$ and/or $R^{3'}$

    or a physiologically tolerated salt thereof.

11. The process as claimed in claim 3 or 4 for the preparation of a pyridine-2,4- or -2,5-dicarboxylic acid amide according to formula I' as claimed in claim 3, in which $R^{1'}$ and $R^{3'}$ are a 3-isopropoxypropyl group and $R^{2'}$ and $R^{4'}$ are hydrogen, or a physiologically tolerated salt thereof.

12. The process as claimed in claim 3 or 4 for the preparation of a pyridine-2,4- or -2,5-dicarboxylic acid amide according to formula I' as claimed in claim 3, in which $R^{1'}$ and $R^{3'}$ are a 2-methoxyethyl group and $R^{2'}$ and $R^{4'}$ are hydrogen, or a physiologically tolerated salt thereof.

**Revendications**

**Revendications pour les Etats contractants suivants : AT, BE, CH, DE, FR, GB, IT, LI, LU, NL, SE**

1. Pyridine-2,4- et -2,5-dicarboxamides de formule I

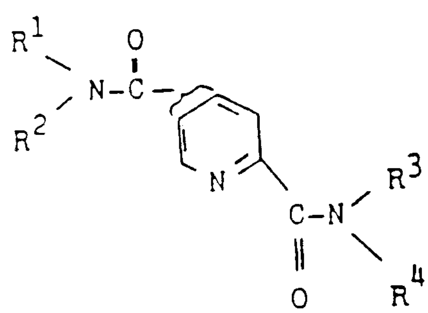

(I)

dans laquelle

$R^1$ représente un radical alkyle en $C_1$-$C_{12}$ ramifié ou non ramifié, qui est éventuellement substitué une fois, ou, dans le cas des radicaux alkyle en $C_2$-$C_{12}$, également plusieurs fois, par

un ou des atomes d'halogène ou groupes hydroxy, cyano, amino, carboxy, alcoxy, alcoxycarbonyle, alkylcarbonyloxy, alkyl- ou dialkylamino, les restes alkyle comportant de 1 à 4 atomes de carbone et, dans le cas des restes alkyle en $C_3$ et $C_4$, pouvant également être ramifiés,

un groupe indolyle ou phényle qui, pour sa part, est éventuellement 1, 2 ou 3 fois substitué par un ou des atomes d'halogène ou radicaux nitro, alkyle en $C_1$-$C_4$ ou alcoxy en $C_1$-$C_4$, dans le cas de substitutions multiples les substituants pouvant également être différents indépendamment les uns des autres, et, dans le cas des radicaux alkyle en $C_3$-$C_4$, ceux-ci pouvant également être ramifiés,

ou $R^1$ est un radical cycloalkyle en $C_5$-$C_7$ saturé qui est éventuellement soudé à un noyau benzénique,

ou $R^1$ est un radical phényle ou naphtyle, ou un cycle aromatique à 5 ou 6 chaînons, comportant 1, 2 ou 3 atomes d'azote et/ou d'oxygène et/ou de soufre, dans chaque cas éventuellement 1, 2 ou 3 fois substitué par un ou des atomes d'halogène ou groupes nitro, cyano, alkyle en $C_1$-$C_4$ ou alcoxy en $C_1$-$C_4$, dans le cas de substitutions multiples les substituants pouvant également être différents indépendamment les uns des autres, et, dans le cas des groupes alkyle en $C_3$-$C_4$, ceux-ci pouvant également être ramifiés,

et

$R^2$ indépendamment de $R^1$ est un atome d'hydrogène ou a l'une des significations données pour $R^1$, $R^2$ pouvant également être identique à $R^1$, et

$R^3$ indépendamment de $R^1$ et $R^2$ est un atome d'hydrogène ou a l'une des significations données pour $R^1$, $R^3$ pouvant également être identique à $R^1$ et/ou $R^2$, et

$R^4$ indépendamment de $R^1$, $R^2$ et $R^3$ est un atome d'hydrogène ou a l'une des significations données pour $R^1$, $R^4$ pouvant également être identique à $R^1$ et/ou $R^2$ et/ou $R^3$, et

les radicaux $R^1$ et $R^2$ et/ou $R^3$ et $R^4$ pouvant également former, conjointement avec l'atome d'azote, un hétérocycle saturé à 5, 6 ou 7 chaînons, l' hétérocycle pouvant également comporter un second atome d'azote, et l' hétérocyclique pouvant pour sa part être substitué par un groupe phényle ou phénylalkyle-($C_1$-$C_3$),

et sels physiologiquement acceptables de ceux-ci, pour utilisation en tant que médicament,
à l'exclusion des composés dans lesquels $R^1$ = $R^3$ et représentent chacun un radical phényle disubstitué par un atome de brome et un groupe méthyle, et $R^2$ = $R^4$ et représentent chacun un atome d'hydrogène.

**2.** Pyridine-2,4- et -2,5-dicarboxamides selon la revendication 1, de formule I, dans laquelle

$R^1$ représente un radical alkyle en $C_1$-$C_{12}$ ramifié ou non ramifié, qui est éventuellement substitué une fois, ou, dans le cas des radicaux alkyle en $C_3$-$C_{12}$, également plusieurs fois, par un ou des groupes amino ou alcoxy en $C_1$-$C_3$, les restes alkyle en $C_3$ pouvant également être ramifiés, et/ou indolyle et/ou phényle, qui peut être substitué par des groupes alcoxy en $C_1$-$C_4$ ou nitro,

ou $R^1$ représente un radical cyclohexyle soudé à un noyau benzénique,

ou $R^1$ représente un radical phényle qui est éventuellement 1, 2 ou 3 fois substitué par un ou des atomes d'halogène ou groupes nitro, cyano ou alkyle en $C_1$-$C_4$, dans le cas de substitutions multiples les substituants pouvant également être indépendamment différents les uns des autres,

ou $R^1$ représente un radical 2-, 3- ou 4-pyridyle, 2- ou 3-thiényle ou thiazolyle, ces radicaux hétéroaromatiques pouvant également être monosubstitués par un groupe alkyle en $C_1$-$C_4$, et

$R^2$ indépendamment de $R^1$ est un atome d'hydrogène ou a l'une des significations données pour $R^1$, $R^2$ pouvant également être identique à $R^1$, et

$R^3$ indépendamment de $R^1$ et $R^2$ est un atome d'hydrogène ou a l'une des significations données pour $R^1$, $R^3$ pouvant également être identique à $R^1$ et/ou $R^2$, et

$R^4$ indépendamment de $R^1$, $R^2$ et $R^3$ est un atome d'hydrogène ou a l'une des significations données pour $R^1$, $R^4$ pouvant également être identique à $R^1$ et/ou $R^2$ et/ou $R^3$,

et sels physiologiquement acceptables de ceux-ci, pour utilisation en tant que médicament, a l'exclusion des composés dans lesquels $R^1$ = $R^3$ et représentent chacun un radical phényle disubstitué par un atome de brome et un groupe méthyle, et $R^2$ = $R^4$ et représentent chacun un atome d'hydrogène.

**3.** Pyridine-2,4- et -2,5-dicarboxamides de formule I'

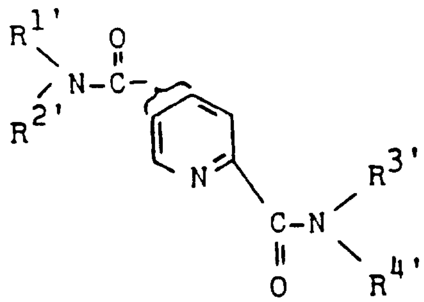

(I')

dans laquelle

$R^{1'}$ représente un radical en $C_1$-$C_{12}$ ramifié ou non ramifié, qui est éventuellement substitué une fois, ou, dans le cas des radicaux alkyle en $C_2$-$C_{12}$, également plusieurs fois, par un ou des atomes d'halogène ou groupes hydroxy, cyano, amino, carboxy, alcoxy, alcoxycarbonyle, alkylcarbonyloxy, alkyl- ou dialkylamino, les restes alkyle comportant de 1 à 4 atomes de carbone, et, dans le cas des restes alkyle en $C_3$ et $C_4$, ceux-ci pouvant également être ramifiés, indolyle ou phényle, qui pour sa part est éventuellement 1, 2 ou 3 fois substitué par un ou des atomes d'halogène ou groupes nitro, alcoxy en $C_1$-$C_4$ ou alkyle en $C_1$-$C_4$, lesdits restes alkyle en $C_3$ et $C_4$ pouvant également être ramifiés et, dans le cas de substitutions multiples, les substituants pouvant également être différents indépendamment les uns des autres,

ou $R^{1'}$ est un radical cycloalkyle en $C_5$-$C_7$ saturé qui est éventuellement soudé à un noyau benzénique,

ou $R^{1'}$ est un radical phényle ou naphtyle, ou un cycle aromatique à 5 ou 6 chaînons, comportant 1, 2 ou 3 atomes d'azote et/ou d'oxygène et/ou de soufre, dans chaque cas éventuellement 1, 2 ou 3 fois substitué par un ou des atomes d'halogène ou groupes nitro, cyano, alkyle en $C_1$-$C_4$ ou alcoxy en $C_1$-$C_4$, dans le cas de substitutions multiples les substituants pouvant également être différents indépendamment les uns des autres, et, dans le cas des groupes alkyle en $C_3$-$C_4$, ceux-ci pouvant également être ramifiés,

$R^{2'}$ indépendamment de $R^{1'}$ est un atome d'hydrogène ou a l'une des significations données pour $R^{1'}$, $R^{2'}$ pouvant également être identique à $R^{1'}$, et

$R^{3'}$ indépendamment de $R^{1'}$ est un atome d'hydrogène ou a l'une des significations données pour $R^{1'}$, $R^{3'}$ pouvant également être identique à $R^{1'}$ et/ou $R^{2'}$, et

$R^{4'}$ indépendamment de $R^{1'}$, $R^{2'}$ et $R^{3'}$ est un atome d'hydrogène ou a l'une des significations données pour $R^{1'}$, $R^{4'}$ pouvant également être identique à $R^{1'}$ et/ou $R^{2'}$ et/ou $R^{3'}$,

et les radicaux $R^{1'}$ et $R^{2'}$ et/ou $R^{3'}$ et $R^{4'}$ pouvant également former, conjointement avec l'atome d'azote, un hétérocycle saturé à 5, 6 ou 7 chaînons,

l'hétérocycle pouvant également comporter un second atome d'azote, et l' hétérocycle pouvant pour sa part être substitué par un groupe phényle ou phénylalkyle($C_1$-$C_3$),

et sels physiologiquement acceptables de ceux-ci, à l'exclusion des composés dans lesquels $R^{1'}$ = $R^{3'}$ et représentent chacun le radical 2-hydroxyéthyle ou 2-(3,4-diméthoxyphényl)éthyle ou un radical phényle disubstitué par un atome de brome et un groupe méthyle, et $R^{2'} = R^{4'}$ et représentent chacun atome d'hydrogène, et du N,N,N',N'-tetracyclohexyl-2,5-pyridinedicarboxamide.

**4.** Pyridine-2,4- et -2,5-dicarboxamides de formule I' selon la revendication 3, dans lesquels

$R^{1'}$ représente un radical alkyle en $C_1$-$C_{12}$ ramifié ou non ramifié, qui est éventuellement substitué une fois, ou, dans le cas de radicaux alkyle en $C_2$-$C_{12}$, également plusieurs fois, par un ou des groupes amino ou alcoxy en $C_1$-$C_3$, les radicaux alkyle comportant de 1 à 3 atomes de carbone et, dans le cas des radicaux alkyle en $C_3$, pouvant également être ramifiés, ou

indolyle ou phényle, qui peut être substitué par un groupe alcoxy en $C_1$-$C_4$ ou nitro,

ou $R^{1'}$ est un radical cyclohexyle soudé à un noyau benzénique,

ou $R^{1'}$ est un radical phényle qui est éventuellement 1, 2 ou 3 fois substitué par un ou des atomes d'halogène ou groupes cyano, alkyle en $C_1$-$C_4$ ou nitro et dans le cas de

substitutions multiples les substituants pouvant également être indépendamment différents les uns des autres,

ou $R^{1'}$ est le radical 2-, 3- ou 4-pyridyle, 3-thiényle ou thiazolyle,

et

$R^{2'}$ indépendamment de $R^{1'}$ est un atome d'hydrogène ou a l'une des significations données pour $R^{1'}$, $R^{2'}$ pouvant également être identique à $R^{1'}$, et

$R^{3'}$ indépendamment de $R^{1'}$ et $R^{2'}$ est un atome d'hydrogène ou a l'une des significations données pour $R^{1'}$, $R^{3'}$ pouvant également être identique à $R^{1'}$ et/ou $R^{2'}$, et

$R^{4'}$ indépendamment de $R^{1'}$, $R^{2'}$ et $R^{3'}$ est un atome d'hydrogène ou a l'une des significations données pour $R^{1'}$, $R^{4'}$ pouvant également être identique à $R^{1'}$ et/ou $R^{2'}$ et/ou $R^{3'}$,

et sels physiologiquement acceptables de ceux-ci, à l'exclusion des composés dans lesquels $R^{1'}$ = $R^{3'}$ et représentent chacun le radical 2-hydroxyéthyle ou 2-(3,4-diméthoxyphényl)éthyle ou un radical phényle disubstitué par un atome de brome et un groupe méthyle, et $R^{2'}$ = $R^{4'}$ et représentent chacun un atome d'hydrogène.

**5.** Procédé pour la préparation de composés de formule I' selon la revendication 3, caractérisé en ce que l'on fait réagir un composé de formule II

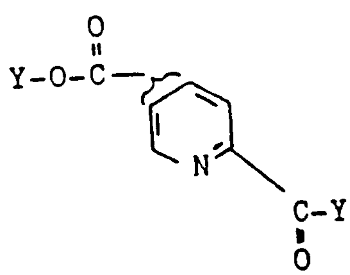

(II)

avec un composé de formule III

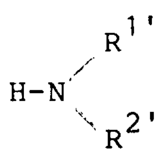

(III)

formules dans lesquelles $R^{1'}$ = $R^{3'}$, $R^{2'}$ = $R^{4'}$ qui ont les significations données à propos de la formule I' et Y est un atome d'halogène ou le groupe hydroxy, ou forme, conjointement avec le groupe carbonyle, un ester actif ou un anhydride mixte, et éventuellement on convertit les produits de réaction en leurs sels physiologiquement acceptables.

**6.** Procédé pour la préparation de composés de formule I' selon la revendication 3, caractérisé en ce que l'on fait réagir un composé de formule II'

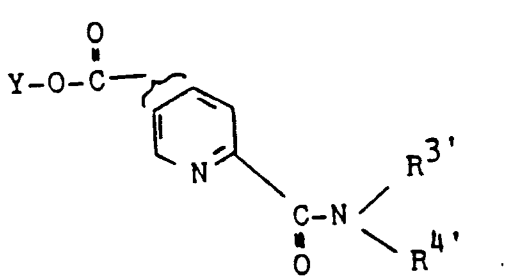

(II')

avec un composé de formule III

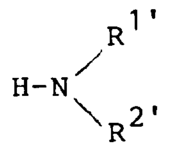

(III)

formules dans lesquelles $R^{1'}$, $R^{2'}$, $R^{3'}$ et $R^{4'}$ ont les significations données à propos de la formule I' et Y est un atome d'halogène ou le groupe hydroxy, ou forme, conjointement avec le groupe carbonyle, un ester actif ou un anhydride mixte, et éventuellement on convertit les produits de réaction en leurs sels physiologiquement acceptables.

**7.** Procédé selon la revendication 5, caractérisé en ce que la réaction s'effectue avec addition simultanée d'un dialkylcarbodiimide, les radicaux dialkyle comportant de 1 à 8 atomes de carbone, et, dans le cas des radicaux en $C_3$-$C_8$,pouvant également être ramifiés ou cycliques.

**8.** Composés selon l'une des revendications 1 à 4, pour l'inhibition de la proline hydroxylase et de la lysine hydroxylase.

**9.** Composés selon l'une des revendications 1 à 4, pour utilisation en tant que fibrosuppresseurs et immunosuppresseurs.

**10.** Médicament contenant un composé de formule I ou I' selon la revendication 1 ou 3, avec des véhicules pharmaceutiquement acceptables.

**11.** Procédé pour la fabrication de médicaments destinés à avoir un effet sur le métabolisme du collagène et de substances similaires au collagène, ou sur la biosynthèse du $C1_q$, caractérisé en ce que l'on met sous une forme d'administration appropriée un composé de formule I ou I' selon la revendication 1 ou 3.

**12.** Pyridine-2,4- et 2,5-dicarboxamides de formule I' selon la revendication 3, dans lesquels

- $R^{1'}$ représente un radical alkyle en $C_1$-$C_4$ ramifié ou non ramifié, qui est substitué par un groupe alcoxy en $C_1$-$C_3$,les restes alkyle comportant de 1 à 3 atomes de carbone, et, dans le cas des composés alkyliques en $C_3$, pouvant également être ramifiés, et
- $R^{2'}$ indépendamment de $R^{1'}$ est un atome d'hydrogène ou a l'une des significations données pour $R^{1'}$, $R^{2'}$ pouvant également être identique à $R^{1'}$, et
- $R^{3'}$ indépendamment de $R^{1'}$ est un atome d'hydrogène ou a l'une des significations données pour $R^{1'}$, $R^{3'}$ pouvant également être identique à $R^{1'}$ et/ou $R^{2'}$, et
- $R^{4'}$ indépendamment de $R^{1'}$, $R^{2'}$ et $R^{3'}$ est un atome d'hydrogène ou a l'une des significations données pour $R^{1'}$, $R^{4'}$ pouvant également être identique à $R^{1'}$, et/ou $R^{2'}$ et/ou $R^{3'}$,

et sels pysiologiquement acceptables de ceux-ci.

**13.** Pyridine-2,4- et -2,5-dicarboxamides de formule I' selon la revendication 3, dans lesquels $R^{1'}$ et $R^{3'}$ sont chacun le groupe 3-isopropoxypropyle, et $R^{2'}$ et $R^{4'}$ sont chacun un atome d'hydrogène, et sels physiologiquement acceptables de ceux-ci.

**14.** Pyridine-2,4- et -2,5-dicarboxamides de formule I' selon la revendication 3, dans lesquels $R^{1'}$ et $R^{3'}$ sont chacun le groupe 2-méthoxyéthyle et $R^{2'}$ et $R^{4'}$ sont chacun un atome d'hydrogène, et sels physiologiquement acceptables de ceux-ci.

**Revendications pour les Etats contractants suivants : ES, GR**

**1.** Procédé pour la fabrication de médicaments, caractérisé en ce que l'on met sous une forme d'administration appropriée des pyridine-2,4- et -2,5-dicarboxamides de formule I

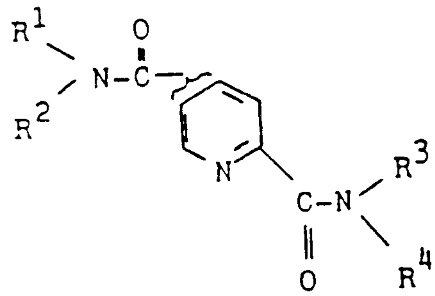

(I)

dans laquelle

$R^1$ représente un radical alkyle en $C_1$-$C_{12}$ ramifié ou non ramifié, qui est éventuellement substitué une fois, ou, dans le cas des radicaux alkyle en $C_2$-$C_{12}$, également plusieurs fois, par

un ou des atomes d'halogène ou groupes hydroxy, cyano, amino, carboxy, alcoxy, alcoxycarbonyle, alkylcarbonyloxy, alkyl- ou dialkylamino, les restes alkyle comportant de 1 à 4 atomes de carbone et, dans le cas des restes alkyle en $C_3$ et $C_4$, pouvant également être ramifiés,

un groupe indolyle ou phényle qui, pour sa part, est éventuellement 1, 2 ou 3 fois substitué par un ou des atomes d'halogène ou radicaux nitro, alkyle en $C_1$-$C_4$ ou alcoxy en $C_1$-$C_4$, dans le cas de substitutions multiples les substituants pouvant également être différents indépendamment les uns des autres, et, dans le cas des radicaux alkyle en $C_3$-$C_4$, ceux-ci pouvant également être ramifiés,

ou $R^1$ est un radical cycloalkyle en $C_5$-$C_7$ saturé qui est éventuellement soudé à un noyau benzénique,

ou $R^1$ est un radical phényle ou naphtyle, ou un cycle aromatique à 5 ou 6 chaînons, comportant 1, 2 ou 3 atomes d'azote et/ou d'oxygène et/ou de soufre, dans chaque cas éventuellement 1, 2 ou 3 fois substitué par un ou des atomes d'halogène ou groupes nitro, cyano, alkyle en $C_1$-$C_4$ ou alcoxy en $C_1$-$C_4$, dans le cas de substitutions multiples les substituants pouvant également être différents indépendamment les uns des autres, et, dans le cas des groupes alkyle en $C_3$-$C_4$, ceux-ci pouvant également être ramifiés,

et

$R^2$ indépendamment de $R^1$ est un atome d'hydrogène ou a l'une des significations données pour $R^1$, $R^2$ pouvant également être identique à $R^1$, et

$R^3$ indépendamment de $R^1$ et $R^2$ est un atome d'hydrogène ou a l'une des significations données pour $R^1$, $R^3$ pouvant également être identique à $R^1$ et/ou $R^2$, et

$R^4$ indépendamment de $R^1$, $R^2$ et $R^3$ est un atome d'hydrogène ou a l'une des significations données pour $R^1$, $R^4$ pouvant également être identique à $R^1$ et/ou $R^2$ et/ou $R^3$,

et les radicaux $R^1$ et $R^2$ et/ou $R^3$ et $R^4$ pouvant également former, conjointement avec l'atome d'azote, un hétérocycle saturé à 5, 6 ou 7 chaînons,

l'hétérocycle pouvant également comporter un second atome d'azote, et l' hétérocycle pouvant pour sa part être substitué par un groupe phényle ou phénylalkyle-($C_1$-$C_3$),

et leurs sels physiologiquement acceptables, à l'exclusion des composés dans lesquels $R^1$ = $R^3$ et représentent chacun un radical phényle disubstitué par un atome de brome et un groupe méthyle, et $R^2$ = $R^4$ et représentent chacun un atome d'hydrogène.

**2.** Procédé pour la fabrication de médicaments, caractérisé en ce que l'on met sous une forme d'administration appropriée des pyridine-2,4- et -2,5-dicarboxamides selon la revendication 1, de formule I, dans lesquels

$R^1$ représente un radical alkyle en $C_1$-$C_{12}$ ramifié ou non ramifié, qui est éventuellement substitué une fois, ou, dans le cas des radicaux alkyle en $C_3$-$C_{12}$, également plusieurs fois, par un ou des groupes amino ou alcoxy en $C_1$-$C_3$, les restes alkyle en $C_3$ pouvant également être ramifiés, et/ou indolyle et/ou phényle, qui peut être substitué par des groupes alcoxy en $C_1$-$C_4$ ou nitro,

ou $R^1$ représente un radical cyclohexyle soudé à un noyau benzénique,

ou $R^1$ représente un radical phényle qui est éventuellement 1, 2 ou 3 fois substitué par

un ou des atomes d'halogène ou groupes nitro, cyano ou alkyle en $C_1$-$C_4$, dans le cas de substitutions multiples les substituants pouvant également être indépendamment différents les uns des autres,

ou $R^1$ représente un radical 2-, 3- ou 4-pyridyle, 2- ou 3-thiényle ou thiazolyle, ces radicaux hétéroaromatiques pouvant également être monosubstitués par un groupe alkyle en $C_1$-$C_4$, et

$R^2$ indépendamment de $R^1$ est un atome d'hydrogène ou a l'une des significations données pour $R^1$, $R^2$ pouvant également être identique à $R^1$, et

$R^3$ indépendamment de $R^1$ et $R^2$ est un atome d'hydrogène ou a l'une des significations données pour $R^1$, $R^3$ pouvant également être identique à $R^1$ et/ou $R^2$, et

$R^4$ indépendamment de $R^1$, $R^2$ et $R^3$ est un atome d'hydrogène ou a l'une des significations données pour $R^1$, $R^4$ pouvant également être identique à $R^1$ et/ou $R^2$ et/ou $R^3$,

et leurs sels physiologiquement acceptables, à l'exclusion des composés dans lesquels $R^1 = R^3$ et représentent chacun un radical phényle disubstitué par un atome de brome et un groupe méthyle, et $R^2 = R^4$ et représentent chacun un atome d'hydrogène.

3. Procédé pour la préparation de pyridine-2,4- et -2,5-dicarboxamides de formule I'

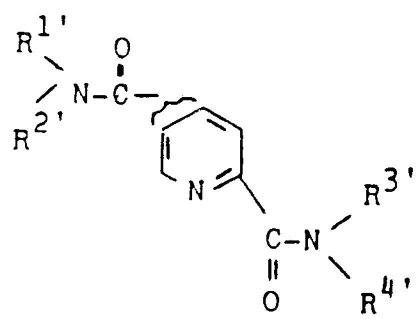

(I')

dans laquelle

$R^{1'}$ représente un radical en $C_1$-$C_{12}$ ramifié ou non ramifié, qui est éventuellement substitué une fois, ou, dans le cas des radicaux alkyle en $C_2$-$C_{12}$, également plusieurs fois, par un ou des atomes d'halogène ou groupes hydroxy, cyano, amino, carboxy, alcoxy, alcoxycarbonyle, alkylcarbonyloxy, alkyl- ou dialkylamino, les restes alkyle comportant de 1 à 4 atomes de carbone, et, dans le cas des restes alkyle en $C_3$ et $C_4$, ceux-ci pouvant également être ramifiés, indolyle ou phényle, qui pour sa part est éventuellement 1, 2 ou 3 fois substitué par un ou des atomes d'halogène ou groupes nitro, alcoxy en $C_1$-$C_4$ ou alkyle en $C_1$-$C_4$, lesdits restes alkyle en $C_3$ et $C_4$ pouvant également être ramifiés et, dans le cas de substitutions multiples, les substituants pouvant également être différents indépendamment les uns des autres,

ou $R^{1'}$ est un radical cycloalkyle en $C_5$-$C_7$ saturé qui est éventuellement soudé à un noyau benzénique,

ou $R^{1'}$ est un radical phényle ou naphtyle, ou un cycle aromatique à 5 ou 6 chaînons, comportant 1, 2 ou 3 atomes d'azote et/ou d'oxygène et/ou de soufre, dans chaque cas éventuellement 1, 2 ou 3 fois substitué par un ou des atomes d'halogène ou groupes nitro, cyano, alkyle en $C_1$-$C_4$ ou alcoxy en $C_1$-$C_4$, dans le cas de substitutions multiples les substituants pouvant également être différents indépendamment les uns des autres, et, dans le cas des groupes alkyle en $C_3$-$C_4$, ceux-ci pouvant également être ramifiés,

$R^{2'}$ indépendamment de $R^{1'}$ est un atome d'hydrogène ou a l'une des significations données pour $R^{1'}$, $R^{2'}$ pouvant également être identique à $R^{1'}$, et

$R^{3'}$ indépendamment de $R^{1'}$ est un atome d'hydrogène ou a l'une des significations données pour $R^{1'}$, $R^{3'}$ pouvant également être identique à $R^{1'}$ et/ou $R^{2'}$, et

$R^{4'}$ indépendamment de $R^{1'}$, $R^{2'}$ et $R^{3'}$ est un atome d'hydrogène ou a l'une des significations données pour $R^{1'}$, $R^{4'}$ pouvant également être identique à $R^{1'}$ et/ou $R^{2'}$ et/ou $R^{3'}$,

et les radicaux $R^{1'}$ et $R^{2'}$ et/ou $R^{3'}$ et $R^{4'}$ pouvant également former, conjointement avec l'atome d'azote, un hétérocycle saturé à 5, 6 ou 7 chaînons,

l'hétérocycle pouvant également comporter un second atome d'azote, et l'hétérocycle

pouvant pour sa part être substitué par un groupe phényle ou phénylalkyle($C_1$-$C_3$), et de leurs sels physiologiquement acceptables, à l'exclusion des composés dans lesquels $R^{1'}$ = $R^{3'}$ et représentent chacun le radical 2-hydroxyéthyle ou 2-(3,4-diméthoxyphényl)-éthyle ou un radical phényle disubstitué par un atome de brome et un groupe méthyle, et $R^{2'}$ = $R^{4'}$ et représentent chacun atome d'hydrogène, et du N,N,N',N'-tétracyclohexyl-2,5-pyridinedicarboxamide, caractérisé en ce que l'on fait réagir un composé de formule II

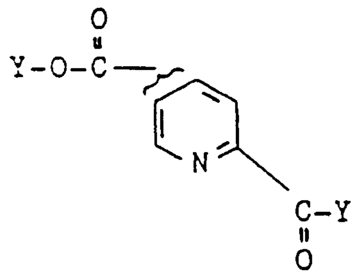

(II)

avec un composé de formule III

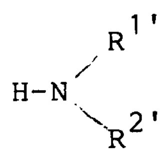

(III)

formules dans lesquelles $R^{1'}$ = $R^{3'}$, $R^{2'}$ = $R^{4'}$ qui ont les significations données à propos de la formule I' et Y est un atome d'halogène ou le groupe hydroxy, ou forme, conjointement avec le groupe carbonyle, un ester actif ou un anhydride mixte, et éventuellement on convertit les produits de réaction en leurs sels physiologiquement acceptables.

**4.** Procédé pour la préparation de composés de formule I' selon la revendication 3, caractérisé en ce que l'on fait réagir un composé de formule II'

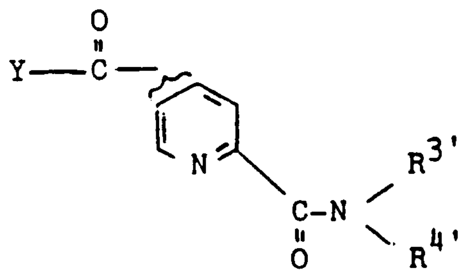

(II')

avec un composé de formule III

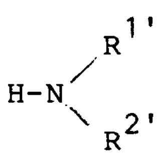

(III)

formules dans lesquelles $R^{1'}$, $R^{2'}$, $R^{3'}$ et $R^{4'}$ ont les significations données à propos de la formule I' et Y est un atome d'halogène ou le groupe hydroxy, ou forme, conjointement avec le groupe carbonyle, un ester actif ou un anhydride mixte, et éventuellement on convertit les produits de réaction en leurs sels physiologiquement acceptables.

**5.** Procédé selon la revendication 3 ou 4, pour la préparation de pyridine-2,4- et -2,5-dicarboxamides de formule I', dans lesquels

$R^{1'}$ représente un radical alkyle en $C_1$-$C_{12}$ ramifié ou non ramifié, qui est éventuellement substitué une fois, ou, dans le cas de radicaux alkyle en $C_2$-$C_{12}$, également plusieurs fois, par un ou des groupes amino ou alcoxy en $C_1$-$C_3$, les radicaux alkyle comportant de 1 à 3 atomes de carbone et, dans le cas des radicaux alkyle en $C_3$, pouvant également être ramifiés, ou

indolyle ou phényle, qui peut être substitué par un groupe alcoxy en $C_1$-$C_4$ ou nitro,

ou $R^{1'}$ est un radical cyclohexyle soudé à un noyau benzénique,

ou $R^{1'}$ est un radical phényle qui est éventuellement 1, 2 ou 3 fois substitué par un ou des atomes d'halogène ou groupes cyano, alkyle en $C_1$-$C_4$ ou nitro et dans le cas de substitutions multiples les substituants pouvant également être indépendamment différents les uns des autres,

ou $R^{1'}$ est le radical 2-, 3- ou 4-pyridyle, 3-thiényle ou thiazolyle,

et

$R^{2'}$ indépendamment de $R^{1'}$ est un atome d'hydrogène ou a l'une des significations données pour $R^{1'}$, $R^{2'}$ pouvant également être identique à $R^{1'}$, et

$R^{3'}$ indépendamment de $R^{1'}$ et $R^{2'}$ est un atome d'hydrogène ou a l'une des significations données pour $R^{1'}$, $R^{3'}$ pouvant également être identique à $R^{1'}$ et/ou $R^{2'}$, et

$R^{4'}$ indépendamment de $R^{1'}$, $R^{2'}$ et $R^{3'}$ est un atome d'hydrogène ou a l'une des significations données pour $R^{1'}$, $R^{4'}$ pouvant également être identique à $R^{1'}$ et/ou $R^{2'}$ et/ou $R^{3'}$,

ainsi que de leurs sels physiologiquement acceptables, à l'exclusion des composés dans lesquels $R^{1'}$ = $R^{3'}$ et représentent chacun le radical 2-hydroxyéthyle ou 2-(3,4-diméthoxyphényl)éthyle ou un radical phényle disubstitué par un atome de brome et un groupe méthyle, et $R^{2'}$ = $R^{4'}$ et représentent chacun un atome d'hydrogène.

**6.** Procédé selon la revendication 3 ou 4, caractérisé en ce que la réaction s'effectue avec addition simultanée d'un dialkylcarbodiimide, les radicaux dialkyle comportant de 1 à 8 atomes de carbone, et, dans le cas des radicaux en $C_3$-$C_8$, pouvant également être ramifiés ou cycliques.

**7.** Procédé pour la fabrication de médicaments destinés à l'inhibition de la proline hydroxylase et de la lysine hydroxylase, caractérisé en ce que l'on met sous une forme d'administration appropriée un composé de formule I ou I' selon la revendication 1 ou 3.

**8.** Procédé pour la fabrication de médicaments pour utilisation en tant que fibrosuppresseurs et immunosuppresseurs, caractérisé en ce que l'on met sous une forme d'administration appropriée un composé de formule I ou I' selon la revendication 1 ou 3.

**9.** Procédé pour la fabrication de médicaments destinés à avoir un effet sur le métabolisme du collagène et de substances similaires au collagène, ou sur la biosynthèse du $C1_q$, caractérisé en ce que l'on met sous une forme d'administration appropriée un composé de formule I ou I' selon la revendication 1 ou 3.

**10.** Procédé selon la revendication 3 ou 4, pour la préparation de pyridine-2,4- et 2,5-dicarboxamides de formule I' selon la revendication 3, dans lesquels

$R^{1'}$ représente un radical alkyle en $C_1$-$C_4$ ramifié ou non ramifié, qui est substitué par un groupe alcoxy en $C_1$-$C_3$,les restes alkyle comportant de 1 à 3 atomes de carbone, et, dans le cas des composés alkyliques en $C_3$, pouvant également être ramifiés, et

$R^{2'}$ indépendamment de $R^{1'}$ est un atome d'hydrogène ou a l'une des significations données pour $R^{1'}$, $R^{2'}$ pouvant également être identique à $R^{1'}$, et

$R^{3'}$ indépendamment de $R^{1'}$ est un atome d'hydrogène ou a l'une des significations données pour $R^{1'}$, $R^{3'}$ pouvant également être identique à $R^{1'}$ et/ou $R^{2'}$, et

$R^{4'}$ indépendamment de $R^{1'}$, $R^{2'}$ et $R^{3'}$ est un atome d'hydrogène ou a l'une des significations données pour $R^{1'}$, $R^{4'}$ pouvant également être identique à $R^{1'}$, et/ou $R^{2'}$ et/ou $R^{3'}$,

ainsi que de leurs sels pysiologiquement acceptables.

**11.** Procédé selon la revendication 3 ou 4, pour la préparation de pyridine-2,4- et -2,5-dicarboxamides de formule I' selon la revendication 3, dans lesquels $R^{1'}$ et $R^{3'}$ sont chacun le groupe 3-isopropoxypropyle,

et $R^{2'}$ et $R^{4'}$ sont chacun un atome d'hydrogène, ainsi que de leurs sels physiologiquement acceptables.

**12.** Procédé selon la revendication 3 ou 4, pour la préparation de pyridine-2,4- et -2,5-dicarboxamides de formule I' selon la revendication 3, dans lesquels $R^{1'}$ et $R^{3'}$ sont chacun le groupe 2-méthoxyéthyle et $R^{2'}$ et $R^{4'}$ sont chacun un atome d'hydrogène, ainsi que de leurs sels physiologiquement acceptables.